(19) Europäisches Patentamt
European Patent Office
Office européen des brevets

(11) Publication number: **0 155 799**
**A2**

(12) **EUROPEAN PATENT APPLICATION**

(21) Application number: 85301535.2

(22) Date of filing: 06.03.85

(51) Int. Cl.[4]: C 07 H 15/207
A 61 K 31/70, C 12 P 19/44
//(C12P19/44, C12R1:465)

(30) Priority: 16.03.84 US 590389

(43) Date of publication of application: 25.09.85 Bulletin 85/39

(84) Designated Contracting States: BE CH DE FR GB IT LI NL SE

(71) Applicant: THE UPJOHN COMPANY
301 Henrietta Street
Kalamazoo, Michigan 49001(US)

(72) Inventor: Argoudelis, Alexander Demetrios
c/o The Upjohn Company 301 Henrietta Street
Kalamazoo Michigan 49001(US)

(72) Inventor: Wiley, Paul Fears
c/o The Upjohn Company 301 Henrietta Street
Kalamazoo Michigan 49001(US)

(72) Inventor: Shilliday, Franklin Barclay
c/o The Upjohn Company 301 Henrietta Street
Kalamazoo Michigan 49001(US)

(72) Inventor: Bannister, Brian
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) **Paulomycin analogues and their preparation.**

(57) New compounds of the formula

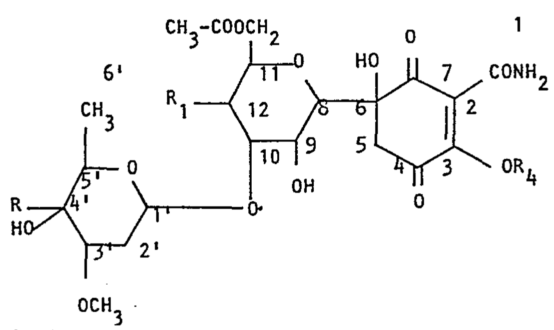

1

(and pharmacologically acceptable salts thereof) wherein R is 1-(alkanoyl)ethyl or acetyl; and $R_1$ is an esterifying group.

EP 0 155 799 A2

# PAULOMYCIN ANALOGUES AND THEIR PREPARATION

The present invention relates to analogues of the antibiotic paulomycin, and to their preparation.

EP-A-0046641 discloses the antibiotics which are named therein as volonomycin A and volonomycin B, but which are now generally known as paulomycin A and paulomycin B. These antibiotics are produced by fermentation of Streptomyces paulus, strain 273, NRRL 12251. Paulomycin, from which the A and B antibiotics can be isolated, is relatively insoluble in water.

EP-A-0124232 discloses antibiotics $273a_1$, $273a_{1\alpha}$ and $273a_{1\beta}$, the first of which is a mixture from which the second and third can be resolved.

Novel compounds according to the present invention are of formula I (and include the pharmacologically acceptable salts thereof)

wherein R is

(a) 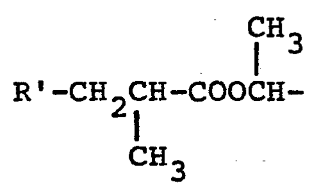

(b) 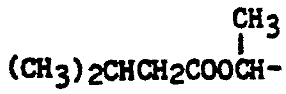

(c) 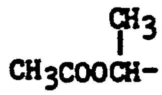

(d) 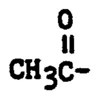

wherein R' is hydrogen or methyl;

wherein $R_1$ is

(a) 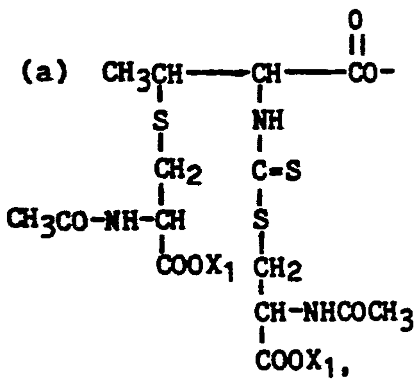

(b) 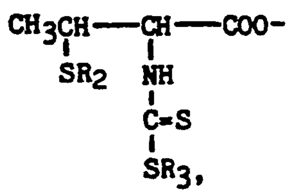

(c) 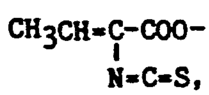 or

(d) 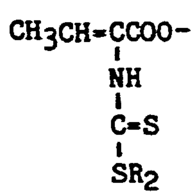

wherein $X_1$ is

(a) hydrogen,

(b) $C_1$ to $C_{12}$ alkyl, branched or straight chain,

(c) a pharmacologically acceptable cation;

wherein $R_2$ and $R_3$ are the same or different and are selected from the group

(a) $-CH_2COOX_2$,

(b) $CH_3CHCOOX_2$,
      |

(c) $COOX_2$
      |
    CH-
      |
    $CH_2$
      |
    $COOX_2$,

(d) $-CH_2CHCOOX_2$
            |
          $NH_2$,

(e) $-CH_2CH_2CHCOOX_2$
                |
              $NHR_5$,

(f) $H_2NCHCH_2CH_2CONHCHCONHCH_2COOX_2$
         |                |
       $COOX_2$         $CH_2-$,

(g) 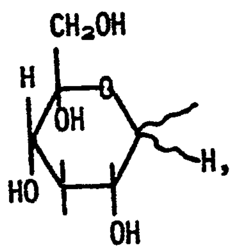

(h) $CH_2-$
      |
    CHOH
      |
    $CH_2OH$,

(i) $CH_2-$
      |
    $[CHOH]_n$
      |
    $CH_2OH$;

(j) $-CH_2CH_2OH$

wherein n is 3 or 4; and

wherein $R_4$ is hydrogen or a pharmacologically acceptable cation;

wherein $R_5$ is hydrogen or acetyl;

wherein $X_2$ is hydrogen or a pharmacologically acceptable cation, with the proviso that when R is

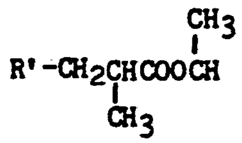

$R_1$ cannot be

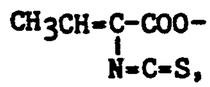, or

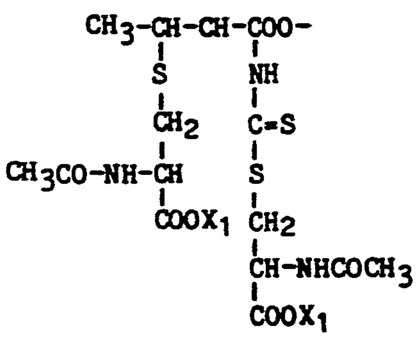

when $X_1$ is hydrogen;

or

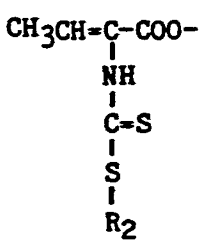

Also provided are:

(a) A process for preparing a compound of Formula Id, a compound of Formula I wherein R is the same as above, and $R_1$ is

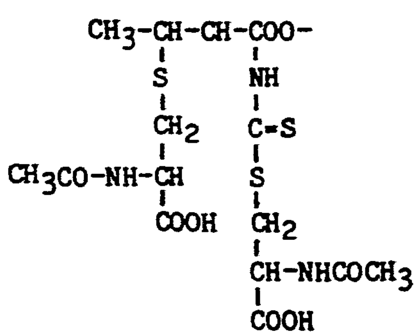

which comprises reacting paulomycin A, $A_2$, B, D or E with N-acetyl-L-cysteine;

(b) A process for preparing a compound of the formula Ia, a compound of Formula I wherein $R_1$ is

(a) 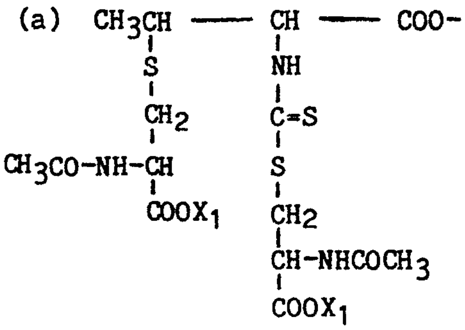

wherein R is

(a) 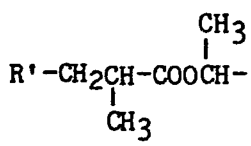

(b) 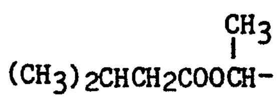

(c) 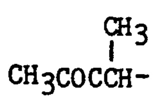

(d) 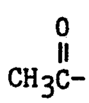

wherein R' is hydrogen or methyl and $X_1$ is $C_1$ to $C_{12}$, alkyl branched or straight chain;

which comprises reacting paulomycin A, $A_2$, B, D, or E with the corresponding esters of N-acetyl-L-cysteine;

(c) A process for preparing a compound of the formula Ib wherein R is

(a) 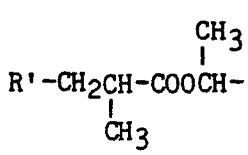

(b) 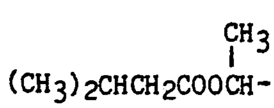

(c) $CH_3$ above: $CH_3COOCH-$

(d) $CH_3\overset{O}{\overset{\|}{C}}-$

wherein R' is hydrogen or methyl;

$R_2$ is

(a) $-CH_2COOH$,

(b) $CH_3CHCOOH$;

(c) 
COOH
|
CH-
|
$CH_2$
|
COOH

(d) $-CH_2CHCOOH$;
with $NHR_5$ on CH

(e) $-CH_2CH_2CHCOOH$;
with $NHR_5$ on CH

(f) $H_2NCHCH_2CH_2CONHCHCONHCH_2COOH$;
with COOH on the first CH and $CH_2-$ on the second CH

(g) 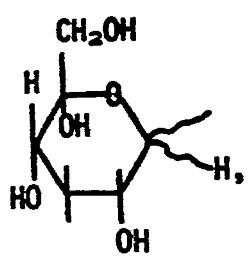 ; or

(h) 
$CH_2-$
|
CHOH
|
$CH_2OH$;

wherein $R_5$ is hydrogen or acetyl;

which comprises reacting paulomycin A, $A_2$, B, D, or E with a compound selected from the following group: mercaptoacetic acid, 2-mercaptopropionic acid, thiomalic acid, cysteine, homocysteine, glutathione, thioglucose and thioglycerol; and

(d) A process for preparing a compound of formula I, a compound of Formula I

wherein R is

$CH_3$
(a) $R'-CH_2CH-COOCH-$
$CH_3$

$CH_3$
(b) $(CH_3)_2CHCH_2COOCH$
$CH_3$

(c) $CH_3COOCH-$

O
(d) $CH_3C-$

wherein R' is hydrogen or methyl;

wherein $R_1$ is $CH_3CH - CHCOO-$
$HOCH_2[CHOH]_nCH_2S$ $NHC=S$
$SCH_2[CHOH]_nCH_2OH$; and

wherein n is 3 or 4;

which comprises reacting paulomycin A, $A_2$, B, D or E with 1-deoxy-1-thiopentitol or 1-deoxy-1-thio-hexitol;

(e) A process for preparing a mixed adduct of formula Ie which comprises reacting a compound having the formula Ib with a compound having the formula

$R_3SH$

wherein R is

$CH_3$
(a) $R'-CH_2CH-COOCH-$

$CH_3$
(b) $(CH_3)_2CHCH_2COOH-$

$CH_3$
(c) $CH_3COOCH-$

(d) $CH_3\overset{O}{\overset{\|}{C}}-$

wherein R' is hydrogen or methyl;

$R_2$ and $R_3$ are different and are selected from the group consisting of

(a) $-CH_2COOX_2$;

(b) $CH_3\underset{|}{C}HCOOH$;

(c) $COOX_2$
$|$
$CH-$
$|$
$CH_2$
$|$
$COOX_2$;

(d) $-CH_2\underset{NHR_5}{\underset{|}{C}}HCOOX_2$

(e) $-CH_2CH_2\underset{NHR_5}{\underset{|}{C}}HCOOX_2$

(f) $H_2N\underset{COOX_2}{\underset{|}{C}}HCH_2CH_2CONH\underset{CH_2-}{\underset{|}{C}}HCONHCH_2COOX_2$,

(g) 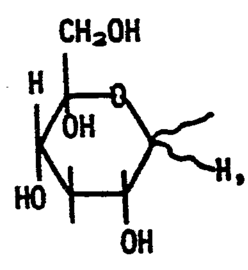

(h) $CH_2-$
$|$
$CHOH$
$|$
$CH_2OH$,

(i) $CH_2-$
$|$
$[CHOH]_n$
$|$
$CH_2OH$; and

(j) $-CH_2CH_2OH$

wherein $R_5$ is hydrogen or acetyl and $X_2$ is hydrogen or a pharmacologically acceptable cation.

Antibiotic $273a_1$ has been discovered in the fermentation broth of Streptomyces paulus which also produces the known antibiotics paulomycin A and paulomycin B. The structures of paulomycin A, $A_2$, B, D and E and antibiotic $273a_1$ are shown in Chart 1 below.

Antibiotics $273a_{1\alpha}$ and $273a_{1\beta}$ can be obtained by reaction of paulomycins A and B, respectively, with N-acetyl-L-cysteine, as disclosed herein. The antibiotics $273a_1$ thus formed, which are tribasic acidic compounds, may then be reacted with appropriate organic or inorganic bases to form mono-, di-, or tri-cation salts. Further, paulomycin A, $A_2$, B, D and E can react with a variety of esters of N-acetyl-L-cysteine, e.g., methyl, ethyl, butyl and octyl, to give antibacterially-active compounds of the general formulas which are shown in Table 1. Still further, paulomycin A, $A_2$, B, D and E when reacted with a variety of compounds, for example, mercaptoacetic acid (5), mercaptopropionic acid (6), thiomalic acid (7), cysteine (8), homocysteine (9), glutathione (10), thioglucose (11), thioglycerol (12), 1-deoxy-1-thio-pentitol (13), and 1-deoxy-1-thio-hexitol (14), shown in Chart II, yield antibacterially-active compounds as shown in Table 1. Salts of the compounds of Table 1 may also be prepared, as noted above and described more fully below. The compounds thus prepared are used in the same manner as the paulomycins.

## DETAILED DESCRIPTION OF THE INVENTION

The present invention is seen more fully by the examples given below.

### Fermentation Procedures

The fermentation procedure described in U.S. Patent 4,335,108, entitled "Paulomycins A and B and Preparation Thereof", except for some difference described below, are used to prepare Paulomycin $A_2$, D and E. The disclosure of U.S. Patent 4,335,108 is hereby expressly incorporated by reference.

### Assay and Testing Procedures

Antibiotic production and purification was measured by a microbiological disc-plate assay procedure with *Micrococcus luteus* as the assay organism.

Thin-layer Chromatographic Procedures

The reaction of paulomycin with N-acetyl-L-cysteine was followed by thin-layer chromatography on silica gel G using chloroform-ethanol-water (25:30:5 v/v) or on cellulose using pH 7.0 phosphate buffer as the solvent systems. The antibiotics present in reaction mixtures in preparations obtained during purification were detected by bioautography on *M. luteus*--seeded trays.

Spectroscopic Methods

Proton magnetic resonance spectra were recorded on a Varian XL-200 spectrometer operating at 200 MHz. Solutions (ca. 0.4 ml, ca. 0.25 M) of the compounds in $d_6$-dimethylsulfoxide or $d_6$-acetone were used. Carbon magnetic resonance spectra were recorded on a Varian CFT-80 spectrometer operating at 20.0 MHz. PMR and CMR chemical shifts are reported as ppm relative to tetramethylsilane. Mass spectra were obtained on a ZAB-2F high resolution mass spectrometer using a fast atom bombardment (FAB) source.

High-Performance Liquid Chromatograph (HPLC)

All HPLC chromatography was carried out on a Hewlett-Packard Model 1084B (Hewlett-Packard, Avondale, California) instrument equipped with an HP model 79875A variable wave length detector and operating in the dual pump mode. A Brownlee 10 cm x 4.6 mm stainless steel column packed with $C_{18}$ (10μ) reverse phase was used. Mobile phases were prepared using Burdick and Jackson distilled in glass solvents. All samples and aqueous phases were filtered through a 0.45 micron filter. The mobile phases used consisted of acetonitrile - pH 5.5 0.1 M phosphate buffer. Samples were prepared as 1 mg/ml solutions in the initial mobile phase. Injection volume was 50 μl.

The following examples are illustrative of the processes and products of the invention, but are not to be construed as limiting. All percentages are by weight and all solvent mixture proportions are by volume unless otherwise noted.

Example 1 Isolation and Characterization of Paulomycin $A_2$

a) Fermentation

The fermentation conditions were in general identical to the conditions described in U.S. Patent 4,335,108. The only exception was that Amberlite XAD-2 resin ca 150 liters, was added per 5000 liters of fermentation. Under these conditions all paulomycins produced are adsorbed on the resin.

(b) Isolation of crude crystalline paulomycins

The whole beer (ca 5000 liters) was adjusted to pH 4.5 (using aqueous sulfuric acid) and then passed through a large vibrating screen in order to remove the Amberlite XAD-2 resin. The resin was slurried in 100 liters of water and screened off on the vibrating screen. The washed resin was packed in a column, washed with 500 liters of cyclohexane-methylene chloride (4:1, v/v) mixture, and then eluted with 1400 liters of ethyl acetate. The ethyl acetate eluate was concentrated to dryness. The dry solid was triturated twice with heptane and then crystallized from methylene chloride. The crude crystalline preparation obtained was found to contain, in addition to paulomycin A and B, several other bioactive components. Further purifica ion was obtained by the procedures described below. The following crude crystalline preparations were used in the recrystallization studies described below.

| Fermentation | Weight of Crude Crystals (g) |
|---|---|
| 148 | 685 |
| 261 | 398 |
| 289 | 785 |
| 366 | 240 |
| 501 | 1324 |
| 383 | 371 |
| 429 | 885 |
| 473 | 589 |

(c) Recrystallization

The crude crystals obtained as described above were dissolved in ethyl acetate (5 ml of ethyl acetate per g of crystals). This solution was mixed with filter aid and Darco G-60 (50 mg of each per g of crude crystals) and stirred for 1 hr. It was then filtered over filter aid and the filtrate was allowed to stand at 5°C for 24 hours. The precipitated crystals of paulomycin A and B were separated to dryness to give a residue (Preparation A) enriched in bioactive materials

other than paulomycins A and B. This material was used for the isolation of paulomycin $A_2$ as described below.

SUMMARY OF RECRYSTALLIZATION

Preparations 148, 161, and 289 were combined and recrystallized from ethyl acetate as described above. The following materials were obtained: 1) 1158 g (paulomycin A and B); 2) Prep 75.2 ca 154 g containing several bioactive materials.

Similarly combination of preparations 366 and 501 and recrystallization yielded 855 g of paulomycins A and B and Preparation 79.3, ca 225 g.

Finally, combination of preparations 383, 429, 473 and recrystallization yielded 1418 g of paulomycins A and B and Preparation 82.2, ca 206 g.

Preparations 79.3 and 82.2 obtained as described above were combined to yield preparation 40.1,431 g, which has been used as the starting material for the work which led to the isolation of paulomycin $A_2$.

(d) Partition Chromatography

Six hundred grams of Dicalite 4200 as mixed with 2800 ml of upper phase and 200 ml of lower phase of the solvent system consisting of dioxane-cyclohexane pH 7.0, 0.1 M phosphate buffer (35:68:8 v/v). The mixture was stirred for 10 minutes. The slurry obtained was introduced into a glass column and packed under 3 Atm pressure to a constant height. The column was then equilibr ted by washing with about 1 L of upper phase of the above solvent.

The starting material, 3.0 g of preparation A containing paulomycin $A_2$ and obtained as described earlier, was dissolved in 15 ml of lower and 10 ml of upper phase. To this solu ion was added 60 g of dicalite and 50 ml of upper phase. The mixture was dried in vacuo. The obtained powder was added on the top of the column and the column was eluted with upper phase. Fractions of 20 ml were collected. Selected fractions were analyzed for bioactivity and by thin layer chromatography. Fractions 95-105 contained mainly paulomycin $A_2$; they were combined, concentrated to one-third of the original volume and freeze dried to give 40 mg of a preparation containing paulomycin $A_2$. Subsequent fractions contained paulomycin A followed by mixture of paulomycins A and B and finally paulomycin B.

Characterization of Paulomycin $A_2$

1. Appearance: Colorless amorphous material

2. Solubility: Soluble in lower alcohols, ketones, ethyl acetate, chloroform, methylene chloride; less soluble in ether; insoluble in saturated hdyrocarbon solvents.

3. Molecular Formula: $C_{34}H_{46}N_2O_{17}S$

4. Molecular Weight: Calcd: 786.2517 Found: 786.522 (by FAB/ms)

5. Anal.Calcd/Found: Calcd for $C_{34}H_{46}N_2O_{17}S$: C,51.90; H,5.85; N,3.56; S,4.07; O, 34.60.

6. $[\alpha]_D^{25}$: -23° (0.4, methanol)

7. Melting Point: 88.5-105°C (decomposition)

8. IRSpectrum: The IR spectrum of paulomycin $A_2$ in Nujol mull and the tabulation of the IR bands follows.

9. The UV spectrum of paulomycin $A_2$ in methanol is as follows:

| λmax(nm) | α | ε |
|---|---|---|
| 237 | 18.66 | 14650 |
| 276 | 12.11 | 9500 |
| 321 | 10.82 | 8500 |

Band Tabulation of the Infrared Spectrum of Paulomycin $A_2$

| Band Freq. | Inten. | Type | | Band Freq. | Inten. | Type | |
|---|---|---|---|---|---|---|---|
| 3468.9 | 65 | SH | | 1189.1 | 41 | AVG | |
| 3370.5 | 57 | BRD | | 1154.3 | 36 | BRD | |
| 3271.2 | 62 | BRD | | 1136.0 | 27 | AVG | |
| 3234.6 | 62 | BRD | | 1119.6 | 26 | AVG | |
| 2949.1 | 1 | SH | M | 1089.4 | 27 | AVG | |
| 2932.7 | 0 | AVG | M | 1055.0 | 32 | AVG | |
| 2870.0 | 6 | AVG | M | 1027.0 | 25 | AVG | |
| 2854.6 | 2 | AVG | M | 998.1 | 40 | SH | |
| 2726.3 | 83 | BRD | M | 992.3 | 40 | AVG | |
| 2689.7 | 85 | SH | | 931.6 | 71 | AVG | |
| 2242.2 | 95 | BRD | | 909.4 | 56 | SHP | |
| 2176.6 | 89 | SH | | 895.9 | 65 | SH | |
| 2118.7 | 66 | SH | | 855.4 | 73 | AVG | |
| 2042.6 | 43 | AVG | | 815.8 | 71 | SHP | |
| 1734.9 | 9 | AVG | | 785.0 | 76 | AVG | |
| 1700.2 | 29 | AVG | | 752.2 | 63 | SHP | |
| 1638.5 | 51 | SH | | 723.3 | 68 | AVG | M |
| 1625.9 | 46 | AVG | | 690.5 | 73 | AVG | |
| 1577.7 | 34 | AVG | | 666.4 | 69 | AVG | |
| 1457.2 | 12 | AVG | M | 637.4 | 69 | SH | |

| | | | | | | |
|---|---|---|---|---|---|---|
| 1377.1 | 19 | AVG | M | 620.1 | 67 | SH |
| 1340.5 | 45 | AVG | | 602.7 | 62 | AVG |
| 1297.1 | 29 | AVG | | | | |
| 1260.4 | 20 | AVG | | | | |

Band Freq.: Band frequencies in wavenumbers (CM-1)

Int.: Intensity in percent transmittance: (%T)

Data type in local peak region: BRD - Broad AVG - Average
SHP - Sharp SH - Shoulder

This peak list is unedited

M: Possible interference from mineral oil

Paulomycin $A_2$

25 Strongest Peaks

| %T | Freq. |
|---|---|
| 0 | 2932.6 |
| 1 | 2949.0 |
| 2 | 2854.5 |
| 6 | 2870.0 |
| 9 | 1734.8 |
| 12 | 1457.1 |
| 19 | 1377.0 |
| 20 | 1260.3 |
| 25 | 1027.0 |
| 26 | 1119.5 |
| 27 | 1136.0 |
| 27 | 1098.3 |
| 29 | 1700.1 |
| 29 | 1297.0 |
| 32 | 1055.0 |
| 34 | 1577.6 |
| 36 | 1154.2 |
| 40 | 998.0 |
| 41 | 1189.0 |
| 43 | 2042.5 |
| 45 | 1340.5 |
| 46 | 1625.8 |
| 51 | 1638.5 |
| 56 | 909.3 |

PAC#: 8357790
Prep: Mineral Oil Mull
Tape: 122 File: 108
Max %T: 97 3750.5
%T at 3800 (CM-1): 96
Density (CM-1/PT): 0.964

Example 2 Isolation and Characterization of Paulomycin D

(a) Fermentation

The fermentation conditions were in general identical to the conditions described in Example 2.

(b) The whole beer (about 500 liters) was adjusted to pH 4.5 (using aqueous sulfuric acid) and then passed through a large vibrating screen in order to remove the Amberlite XAD-2 resin. The resin was slurried in 100 liters of water and screened off on the vibrating screen. The washed resin was packed in a column, washed with 500 liters of cyclohexane-methylene chloride (4:1, v/v) mixture, and then eluted with 1400 liters of ethyl acetate. The ethyl acetate eluate was concentrated to dryness. The dry solid was triturated twice with heptane and then crystallized from methylene chloride. The crude crystalline preparation obtained was found to contain, in addition to paulomycin A and B, several other bioactive components. Further purification was obtained by the procedures described below.

Crude crystalline preparations were used in the recrystallization studies described below.

| Fermentation | Weight of Crude Crystals (g) |
|---|---|
| 148 | 685 |
| 261 | 398 |
| 289 | 785 |
| 366 | 240 |
| 501 | 1324 |
| 383 | 371 |
| 429 | 885 |
| 473 | 589 |

(c) Recrystallization

The crude crystals obtained as described above were dissolved in ethyl acetate (5 ml of ethyl acetate per g of crystals). This solution was mixed with filter aid and Darco G-60(50 mg of each per g of crude crystals) and stirred for 1 hr. It was then filtered over filter aid and the filtrate was mixed with hexane (5 ml per g crude crystals). The mixture was allowed to stand at 5°C for 24 hours. The precipitated crystals of paulomycin A and B were separated by filtration. The filtrate (mother liquors) was concentrated to dryness to give a residue (Preparation A) enriched in bioactive materials

other than paulomycins A and B. This material was used for the isolation of paulomycin D as described below.

Summary of Recrystallization

Preparations 148, 261 and 289 were combined and recrystallized from ethyl acetate as described above. The following materials were obtained: 1) 1158 g (paulomycin A and B); 1) Preparation 75.2, ca 154 g, containing several bioactive materials).

Similarly combination of preparations 366 and 501 and recrystallization yielded 855 g of paulomycins A and B and Preparation 79.3 (about 225 g).

Finally, combination of preparations 383, 429, 473 and recrystallization yielded 1418 g of paulomycins A and B and Preparation 82.2 (about 206 g).

Preparations 79.3 and 82.2 obtained as described above were combined to yield preparation 40.1, 431g, which has been used as the starting material for the work which led to the isolation of paulomycin D.

(d) First HPLC (Isolation of "polar paulomycin")

Instrument: Waters Prep 500A

Support: Waters C-18 Reverse Phase Silica Packed Columns

Starting Material: Prep 40.1, 20.0 g

Mobile Phase: Acetonitrile-0.1M, pH 5.5 phosphate buffer (1:1 v/v)

Flow Rate: 200 ml/minute

The starting material was dissolved in 400 ml of acetonitrile-pH 5.5 phosphate buffer (1:1 v/v) and was injected into the column. The chromatography was followed by UV and refractive index detectors. In addition, selected fractions were analyzed by TLC using KC-18 reverse phase silica plates and acetonitrile-methanol-pH 5.5, 0.01 M phosphate buffer (1:1:1, v/v) as the mobile phase. Seven fractions were collected and and characterized as follows:

Fraction 1 = Polar Paulomycins (including Paulomycin D)
Fraction 2 = Paulomycin B
Fraction 3 = Paulomycin B,A
Fraction 4 = Paulomycin A
Fraction 5 = Paulomycin A (Tail-Fraction I)
Fraction 6 = Paulomycin A (tail-Fraction II)
Fraction 7 = Methanolic wash

A total of 12 runs were made using a total of 250 g of prep 40.1. Similar fractions from all 12 runs were combined and extracted twice with methylene chloride. The combined methylene chloride extracts from Fraction 1 were concentrated to dryness to give preparation D 14:16. This material was used for isolation of purified paulomycin D as described below.

(e) Second HPLC

Instrument: Waters Prep 500A

Support: Waters C-18 Reverse Phase Silica Packed Columns

Starting Material: 14:16

Mobile Phase: Acetonitrile-pH 5.5, 0.1M, pH 5.5 phosphate buffer (40:60, v/v)

Flow Rate: 200 ml/minute

The starting material was dissolved in about 400 ml of acetonitrile-pH 5.5,0.1M phosphate buffer (1:1, v/v) and the solution was introduced into the column. The chromatography was followed by UV and refractive index detectors and by TLC using the system described above. Seven fractions were collected and designated as follows:

| | |
|---|---|
| Fraction 1 | = Non-designated materials |
| Fraction 2 | = Paulomycin G |
| Fraction 3 | = Paulomycins G,F |
| Fraction 4 | = Paulomycins F,E |
| Fraction 5 | = Paulomycins E |
| Fraction 6 | = Paulomycins E,D |
| Fraction 7 | = Paulomycins D,C |

A total of 4 runs were run using all of preparation 14;16. Similar fractions from all 4 runs were combined and extracted twice with methylene chloride. The combined methylene chloride extracts from fraction 6 (paulomycins E, D) were concentrated to dryness to give preparation 20.6, 4.98 g. Preparation 20.6 contained mainly paulomycin D and was purified further as described below.

<u>Characterization of Paulomycin D</u>

1. Appearance: Colorless amorphous material

2. Solubility: Soluble in lower alcohols, ketones, ethyl acetate, chloroform, methylene chloride; less soluble in ether; insoluble in saturated hdyrocarbon solvents.

3. Molecular Formula: $C_{31}H_{40}N_2O_{17}S$

4. Calcd Mol Weight: 744.20475 Found: (HR-FAB/MS): 744.20483

5. Anal.Calcd/Found: Calcd for $C_{31}H_{40}N_{2}O_{17}S$: C, 50.00; H, 5.37; N, 3.76; S, 4.30; O, 36.56.

6. $[\alpha]_D^{25}$: -33° (c, 0.8, methanol)

7. Melting Point: Decomposition at about 140°C

8. IR Spectrum: The IR spectrum of paulomycin D in Nujol mull and tabulation of the IR bands follows.

9. UV Spectrum: The UV Spectrum of paulomycin D is as follows in Figure 3.

| λmax(nm) | α | ε |
|---|---|---|
| 241 | 19.66 | 14600 |
| 276 | 14.17 | 10550 |
| 321 | 11.43 | 8500 |

Band tabulation of the infrared spectrum of paulomycin D

| Band Freq. | Inten. | Type | Band Freq. | Inten. | Type |
|---|---|---|---|---|---|
| 3473.7 | 59 | SH | 1257.5 | 13 | AVG |
| 3374.4 | 50 | BRD | 1200.6 | 45 | BRD |
| 3272.2 | 55 | AVG | 1191.0 | 44 | AVG |
| 3234.6 | 56 | BRD | 1153.4 | 31 | SH |
| 2954.9 | 0 | AVG M | 1136.0 | 23 | AVG |
| 2908.6 | 0 | BRD M | 1117.7 | 23 | AVG |
| 2868.1 | 3 | SH M | 1098.4 | 24 | AVG |
| 2854.6 | 1 | AVG M | 1056.0 | 24 | AVG |
| 2725.4 | 79 | BRD M | 1029.0 | 19 | AVG |
| 2668.5 | 81 | BRD M | 996.2 | 31 | AVG |
| 2177.6 | 87 | SH | 930.6 | 64 | AVG |
| 2119.7 | 65 | SH | 910.3 | 50 | SHP |
| 2043.5 | 44 | AVG | 896.8 | 59 | SH |
| 1734.9 | 5 | AVG | 855.4 | 66 | AVG |
| 1700.2 | 23 | AVG | 816.8 | 66 | SHP |
| 1625.9 | 37 | AVG | 783.0 | 70 | AVG |
| 1576.8 | 27 | AVG | 752.2 | 57 | SHP |
| 1458.1 | 6 | AVG M | 723.3 | 60 | AVG M |
| 1377.1 | 11 | AVG M | 690.5 | 64 | AVG |
| 1342.4 | 38 | AVG | 654.8 | 58 | AVG |
| 1298.0 | 26 | AVG | | | |

Band Freq.: Band frequencies in wavenumbers (CM-1)

Int.: Intensity in percent transmittance: (%T)

Data type in local peak region: BRD - Broad AVG - Average

SHP - Sharp SH - Shoulder

This peak list is unedited

M: Possible interference from mineral oil

Paulomycin D

25 Strongest Peaks

| %T | Freq. |
|---|---|
| 0 | 2954.8 |
| 0 | 2908.5 |
| 1 | 2854.5 |
| 3 | 2868.0 |
| 5 | 1734.8 |
| 6 | 1458.0 |
| 11 | 1377.0 |
| 13 | 1257.5 |
| 19 | 1029.0 |
| 23 | 1700.1 |
| 23 | 1136.0 |
| 23 | 1117.6 |
| 24 | 1098.3 |
| 24 | 1056.0 |
| 26 | 1298.0 |
| 27 | 1576.7 |
| 31 | 1153.3 |
| 31 | 996.1 |
| 37 | 1625.8 |
| 38 | 1342.3 |
| 44 | 2043.5 |
| 44 | 1192.0 |
| 45 | 1200.5 |
| 50 | 3374.3 |
| 50 | 910.2 |

PAC#: 8459425
Prep: Mineral Oil Mull
Tape: 122 File: 109
Max %T: 99 3749.6
%T at 3800 (CM-1): 99
Density (CM-1/PT): 0.964

Example 3 Isolation and Characterization of Paulomycin E

(a) Fermentation

The fermentation procedures described in Example 2 were used.

(b) Isolation of crude solids

The fermentation broth, about 4700 liters, was adjusted to pH 3.0 with sulfuric acid, cooled to 18°C and filtered with the aid of diatomaceous earth. The filtered beer was discarded. The filter cake was slurried with 2000 liters of ethyl acetate and the mixture was then filtered through a filter press. The filtrate (ethyl acetate extract containing paulomycins) was concentrated to a volume of about 110 liters. This concentrate was mixed with 30 kg of Harbolite 2000 filter aid (Harbolite Co.) and the wet mixture was dried in vacuo at 40°C. (Reference 149B; 152C).

The dried Harbolite-paulomycin mixture, obtained as described above, was poured into a column partially filled with heptane and was packed under atmospheric pressure. The column was eluted at a rate of 1.5 liters per minute. The following fractions were collected.

| | |
|---|---|
| 1 | Heptane, 720L |
| 2 | Heptane-ethyl acetate 97:3 (v/v), 180L |
| 3 | Heptane-ethyl acetate 94:6 (v/v), 180L |
| 4 | Heptane-ethyl acetate 91:9 (v/v), 180L |
| 5 | Heptane-ethyl acetate 88:12 (v/v), 180L |
| 6 | Heptane-ethyl acetate 85:10 (v/v), 180L |
| 7 | Heptane-ethyl acetate 85:10 (v/v), 180L |
| 8 | Heptane-ethyl acetate 85:10 (v/v), 180L |
| 9 | Heptane-ethyl acetate 85:10 (v/v), 180L |

Fractions 4, 5, and 6 containing most of the bioactivity were concentrated to dryness in vacuo. Fraction 4 gave preparation 156A, 35.2g; Fraction 5 gave preparation 156B, 66.4 g; and Fraction 6 gave preparation 156C, 66.3g.

(c) Isolation of Crude Crystalline Paulomycins

Preparation 156B was dissolved in 166 liters of chloroform. Ethyl ether (500 ml) and heptane (4 liters) was added and the mixture was allowed to stand at 5°C for 20 hours. Crystalline paulomycin containing paulomycin E in addition to paulomycins A and B was isolated by filtration and dried to give preparation 8.1, 20.3 g).

Preparation 156C was treated in a similar way to give prep 9.1, 24 g.

(d) HPLC

Dupont Zorbax RP-18 silica, 1.5 kg was slurried in 2 liters of 0.1 M potassium phosphate buffer, pH 5.5 acetonitrile (60:40, v/v). The silica was packed under pressure in the ISA-Prep 100 HPLC machine. The column was equilibrated by passing 3 liters of the above solvent over it.

Crude crystalline paulomycin (25 g) obtained in C, was dissolved in the solvent and introduced into the column. The column was eluted at a rate of 20 ml/min and monitored using a UV detector at 323 nm. Fractions eluted from the column were analyzed by thin layer chromatography. Fractions containing paulomycin E were combined to give preparation 32.2, 325 mg.

(e) Isolation of Pure Paulomycin E

Preparation 32.3 was dissolved in 10 ml of chloroform. The solution was clarified by filtration and mixed with 50 ml of ether. Insoluble material was isolated by filtration. The filtrate was mixed with 100 ml of heptane. The precipitated paulomycin E was isolated by filtration and dried (350 mg).

Characterization of Paulomycin E

(Prep. ADA-136.2)

1. Appearance: Colorless amorphous material

2. Solubility: Soluble in lower alcohols, ketones, ethyl acetate, chloroform, methylene chloride; less soluble in ether; insoluble in saturated hdyrocarbon solvents.

3. Molecular Formula: $C_{29}H_{36}N_2O_{16}S$

4. Molecular Weight: Calcd 700.17854 Found: 786.2522 (by FAB/MS)

5. Anal.Calcd/Found: Calcd for $C_{29}H_{36}N_2O_{16}S$: C, 49.71; H, 5.14; N, 4.00; S, 4.57; O, 36.57.

6. $[\alpha]_D^{25}$: -26° (c, 0.78, methanol)

7. Melting Point: Decomposition over a broad range (120-160°C)

8. IR Spectrum: The IR spectrum of paulomycin E in Nujol mull and tabluation of the IR bands follows:

11. UV Spectrum: The UV Spectrum of paulomycin E is as follows in Figure 3.

| λmax(nm) | α | ε |
|---|---|---|
| 240 | 19.31 | 13500 |
| 276 | 13.16 | 9200 |
| 322 | 12.81 | 8950 |

Band tabulation of the infrared spectrum of paulomycin E

| Band Freq. | Inten. | Type | Band Freq. | Inten. | Type |
|---|---|---|---|---|---|
| 3625.2 | 89 | SH | 1300.0 | 17 | AVG |
| 3468.0 | 51 | SH | 1259.5 | 10 | AVG |
| 3382.1 | 42 | BRD | 1245.0 | 13 | SH |
| 3269.3 | 49 | BRD | 1199.7 | 41 | AVG |
| 3234.6 | 49 | BRD | 1157.2 | 24 | AVG |
| 2959.7 | 0 | AVG M | 1137.0 | 17 | AVG |
| 2868.1 | 1 | SH M | 1111.9 | 14 | AVG |
| 2853.6 | 0 | AVG M | 1055.0 | 23 | AVG |
| 2856.5 | 0 | AVG M | 1028.0 | 11 | AVG |
| 2727.3 | 75 | AVG M | 997.1 | 24 | AVG |
| 2671.4 | 77 | BRD N | 972.1 | 50 | SH |
| 2424.5 | 92 | BRD | 910.3 | 41 | SHP |
| 2245.1 | 92 | BRD | 894.0 | 52 | AVG |
| 2177.6 | 83 | SH | 855.4 | 65 | AVG |
| 2118.7 | 54 | SH | 816.8 | 61 | SHP |
| 2043.5 | 33 | AVG | 784.0 | 66 | AVG |
| 1882.5 | 95 | BRD | 751.2 | 48 | SHP |
| 1735.9 | 4 | AVG | 723.3 | 56 | AVG M |
| 1707.9 | 12 | AVG | 690.5 | 61 | AVG |
| 1625.9 | 27 | AVG | 667.3 | 58 | AVG |
| 1576.8 | 17 | AVG | 636.5 | 59 | SH |
| 1457.2 | 3 | AVG M | 600.8 | 50 | AVG |
| 1377.1 | 7 | AVG M | | | |
| 1343.4 | 29 | SH | | | |

Band Freq.: Band frequencies in wavenumbers (CM-1)

Int.: Intensity in percent transmittance: (%T)

Data type in local peak region: BRD - Broad AVG - Average
SHP - Sharp SH - Shoulder

This peak list is unedited

M: Possible interference from mineral oil

Paulomycin E

25 Strongest Peaks

| %T | Freq. |
|---|---|
| 0 | 2959.6 |
| 0 | 2853.5 |
| 0 | 2856.5 |
| 1 | 2868.0 |
| 3 | 1457.1 |
| 4 | 1735.8 |
| 7 | 1377.0 |
| 10 | 1259.5 |
| 11 | 1028.0 |
| 12 | 1707.8 |
| 13 | 1245.0 |
| 14 | 1111.8 |
| 17 | 1576.6 |
| 17 | 1300.0 |
| 17 | 1137.0 |
| 23 | 1055.0 |
| 24 | 1157.1 |
| 24 | 997.0 |
| 27 | 1625.8 |
| 29 | 1343.3 |
| 33 | 2043.5 |
| 41 | 1199.6 |
| 41 | 910.2 |
| 42 | 3382.0 |
| 48 | 751.1 |

PAC#: 8459043
Prep: Mineral Oil Mull
Tape: 122 File: 107
Max %T: 97 3754.4
%T at 3800 (CM-1): 97
Density (CM-1/PT): 0.964

Example 4 Preparation of Antibiotic $273a_1$

N-Acetyl-L-cysteine, 25.2 g, was dissolved in 500 ml of 0.1 M pH 7.85 phosphate buffer. This solution was adjusted to pH 8.7 with 1N aqueous potassium hydroxide. Paulomycin (A and B mixture), 5.98 g (prepared as described in U.S. patent 4,335,108, which is expressly incorporated by reference herein), was added to this solution and dissolved under stirring. After standing at room temperature for 1 hours, the solution was adjusted to pH 3.0 with 2N aqueous hydrochloric acid and extracted three times with 500 ml portions of ethyl acetate. The ethyl acetate extracts were combined, dried over sodium sulfate and concentrated to dryness to give prep. -151.1, 14.0 g. Preparation -151.1 contained antibiotic $273a_1$ as the only bioactive component. Thin layer chromatography on silica gel using chloroform-ethanol-water (25:35:5 v/v) as the mobile phase showed the presence of antibiotic $273a_1$ and N-acetyl-L-cysteine as the only materials present when the plates were developed with $MnO_4K$-$IO_4K$ spray reagent. Preparation - 151.1 was found highly active vs. M. luteus ca. 256 bu/mg.

Prep. -151.1 was dissolved in 120 ml of acetone; this solution was added under stirring to 1.1 L of ether. The precipitated material was isolated by filtration, dried on the filter, and redissolved in 120 ml of acetone. The new solution was also added to 1.1 L of ether. The precipitated material was isolated by filtration and dried (prep. -152.1 6.2 g). The two filtrates (filtrate I and filtrate II) from the above precipitations gave, by concentration to dryness, preparations -152.2 and -152.3, respectively.

Preparation -152.1 was found to contain by TLC and HPLC antibiotics $273a_{1\alpha}$ and $273a_{1\beta}$. Preparation -152.2 contained N-acetyl-L-cysteine. Preparation -152.3 contained N-acetylcysteine, small amounts of antibiotics $273a_1$ and traces of an unidentified material.

Example 5 Preparation of Antibiotic $273a_{1\alpha}$

N-Acetyl-L-cysteine, 4.2 g, was dissolved in 100 ml of 0.1 M pH 7.85 phosphate buffer. The solution was adjusted to pH 8.7 with 1N aqueous potassium hydroxide. Paulomycin A, 1.0 g was added to this solution and dissolved under stirring. After standing at room temperature for 1 hour, the solution was adjusted to pH 3.0 with 2N aqueous hydrochloric acid and extracted four times with 100 ml portions

of ethyl acetate. The ethyl acetate extracts were combined, dried over sodium sulfate and concentrated to dryness to give prep. - 144.1. Preparation - 144.1 was found highly active vs. M. luteus, ca. 300 bu/mg and contained antibiotic 273a$_1$α as the only bioactive component.

Prep. -144.1 was dissolved in 15 ml of acetone; this solution was added under stirring to 200 ml of ether. The precipitated material was isolated by filtration, dried on the filter, and redissolved in 15 ml of acetone. The new solution was also poured into 200 ml of ether under stirring. The precipitate formed was isolated by filtration and dried (prep. -145.1, 910 mg). The two filtrates (filtrate I and filtrate II) from the above precipitations gave, by concentration to dryness, preparations -145.2 and -145.3, respectively.

Preparation -145.1 contained by TLC and HPLC antibiotic 273a$_1$α. Preparation -145.2 contained N-acetyl-L-cysteine. Preparation -145.3 contained N-acetyl-L-cysteine, small amounts of antibiotic 273a$_1$α and traces of a polar unidentified material.

Example 6 Preparation of Antibiotic 273a$_1$β

N-Acetyl-L-cysteine, 4.2 g, was dissolved in 100 ml of 0.1 M pH 7.85 phosphate buffer. The solution was adjusted to pH 8.7 with 1 N aqueous potassium hydroxide. Paulomycin B, 1.0 g, was added to this solution and dissolved under stirring. After standing at room temperature for 1 hour, the solution was adjusted to pH 3.0 with 2N aqueous hydrochloric acid and extracted four times with 100 ml portions of ethyl acetate. The ethyl acetate extracts were combined, dried over sodium sulfate and concentrated to dryness to give prep. -146.1. Preparation -146.1 was found highly active vs. M. luteus, ca. 300 by/mg and contained antibiotic 273a$_1$β as the only bioactive component.

Prep. -144.1 was dissolved in 15 ml of acetone; this solution was added under stirring to 200 ml of ether. The precipitated material was isolated by filtration, dried on the filter, and redissolved in 15 ml of acetone. The new solution was also poured into 200 ml of ether under stirring. The precipitate formed was isolated by filtration and dried (prep. -147.1, 111 g). The two filtrates (filtrate I and filtrate II) from the above precipitations gave, by concentration to dryness, preparations -147.2 and -147.3, respectively.

Preparation -147.1 contained by TLC and HPLC antibiotic $273a_1\beta$. Preparation -147.2 contained N-acetyl-L-cysteine. Preparation -147.3 contained N-acetyl-L-cysteine, small amounts of antibiotic $273a_1\beta$ and an unidentified polar compound.

Utilizing a procedure similar to that described in Examples 5 and 6 but substituting paulomycin $A_2$, D and E for paulomycins A and B, there is obtained the antibiotic analogs of paulomycin $A_2$. D and E that correspond to antibiotics $273a_1\alpha$ and $273a_1\beta$.

Example 7 Reaction of Paulomycins A, $A_2$, B, D and E with Esters of N-Acetyl-L-Cysteine

1. Preparation of N-Acetyl-L-Cysteine Methyl, Ethyl, Butyl, and Octyl Esters.

N-Acetyl-L-cysteine (1 equivalent) is dissolved in excess of methyl, ethyl, butyl, or octyl alcohol. Thionyl chloride (1.1 to 1.5 equivalents) is then added to the N-acetyl-L-cysteine-alcohol mixture and this mixture is allowed to stand at room temperature for 1-3 hours. The reaction mixture is then concentrated to dryness. Crystallization of the desired product is obtained from ether-heptane mixture.

2. Reaction of Paulomycins (A, $A_2$, B, D or E) with N-Acetyl-L-Cysteine Methyl, Ethyl, Butyl and Octyl Esters

Paulomycin (1 equivalent) is dissolved in tetrahydrofuran containing catalytic amounts of triethylamine. Ten equivalents of the corresponding N-acetyl-L-cysteine (methyl, ethyl, butyl, or octyl ester) is then added under stirring. After 30 minutes at room temperature the reaction mixture is concentrated to dryness. The residue is then dissolved in methylene chloride and this solution is mixed with Skellysolve B. The paulomycin-N-acetyl-L-cysteine ester adducts precipitate and are isolated by filtration and dried. Characterization is obtained by fast atom bombardment mass spectrometry.

Example 8 Reaction of Paulomycins (A, $A_2$, B, D and E) with Mercaptoacetic Acid (5), Mercaptopropionic Acid (6) and Thiomalic Acid (7)

Compounds 5, 6, and 7 were obtained from suppliers like Sigma Co., Aldrich Co., and Alfa Co.

Reaction of Paulomycins with Mercaptoacetic Acid (5)

Mercaptoacetic acid (20 eq) is dissolved in pH 8.5 phosphate buffer. The pH was then adjusted to 8.7 with 1N KOH and 1 equivalent of paulomycin A, $A_2$, B, D or E is added under stirring. After 2 hours at room temperature under stirring the solution is adjusted to pH 3.0 and the paulomycins-mercaptoacetic acid addition compound is isolated by extraction with methylene chloride or ethyl acetate. The extract is dried over sodium sulfate and concentrated to dryness. The residue obtained is then purified by precipitations from methylene chloride-heptane combinations and the precipitate is characterized by fast atom bombardment mass spectrometry.

Example 9 Reaction of Paulomycin (A, $A_2$, B, D, or E) with Mercaptopropionic Acid (6) and Thiomalic Acid (7)

The procedure described in Example 5 is followed and specifically the ratio of the acids to paulomycin was about 20:1 equivalents. The precipitates obtained by the methylene chloride precipitation were analyzed by TLC, HPLC, and fast atom bombardment mass spectroscopy.

Example 10 Reaction of Paulomycins A, $A_2$, B, D and E with Cysteine (8), Homocysteine (9) and Glutathione (10)

The above compounds 8, 9 and 10 were obtained from different suppliers of chemicals like Sigma Co., Aldrich Co., and Alfa Co.

1. Reaction with Cysteine(8)

Cysteine hydrochloride (439 mg, 2.5 mmoles) is dissolved in 20 ml of pH 7.85 0.1 M phosphate buffer. The pH of the solution was adjusted to 8.7 with 1N potassium hydroxide. Paulomycin (a mixture of paulomycins A and B), 200 mg (0.25 mmoles) was added under stirring. The reaction was followed by thin layer chromatography. The same procedure can be utilized substituting paulomycins A, $A_2$, B, D or E for the mixture of paulomycins A and B.

2. Reaction with Homocysteine (9)

The procedure described above was followed using 337.5 mg of homocysteine (2.5 mmoles) and 200 mg of paulomycin (mixture of paulomycins A and B). Likewise, the procedure can be utilized substituting paulomycins A, $A_2$, B, D and E for the mixture of paulomycins A and B.

3. Reaction with Glutathione (10)

The procedure described above was followed using 767.5 mg of glutathione (2.5 mmoles) and 200 mg of paulomycin (mixture of paulomycin A and B). Paulomycins A, $A_2$, B, D and E can be substituted for the mixture of paulomycins A and B.

4. <u>Purification of Reaction Products</u>

The products of the reactions of paulomycins A, $A_2$, B, D and E and cysteine, homocysteine and glutathione can be extracted by butanol at pH 6.0. The butanolic extract is concentrated to dryness and the residue is then purified by chromatography over silica gel using chloroform-methanol mixtures as the mobile phase. Alternatively, the residue obtained from the butanolic extract can be purified by reverse phase chromatography using C-18 or C-8 silica and acetonitrile-pH 5.5 1 M phosphate buffer mixtures.

The products of the reactions of paulomycin with cysteine, homocysteine, and glutathione were characterized by fast atom bombardment mass spectrometry.

The products of the reactions of paulomycin with cysteine, homocysteine, and glutathione were characterized by fast atom bombardment mass spectrometry.

<u>Example 11</u> Reaction of Paulomycin A, $A_2$, B, D and E with Thioglucose (11) or Thioglycerol (12)

Thioglucose (11) or thioglycerol (12) (20 eq) were dissolved in pH 7.85 phosphate buffer. The pH was then adjusted to 8.7 with 1N KOH solution and 1 equivalent of paulomycin (A or B as mixture) was added under stirring. After 1 hour at room temperature the solution was adjusted to pH 5.5 and passed over Amberlite XAD-4. The paulomycin-thioglucose or thioglycerol reaction products were absorbed on the resin and eluted with acetone. The acetone solution was concentrated to dryness. The residue was dissolved in acetone or methylene chloride and this solution was mixed with ether-hexane mixture. The precipitated paulomycin-thioglucose or paulomycin-thioglycerol product was isolated by filtration and dried. Characterization of these materials was obtained by fast atom bombardment mass spectroscopy.

Likewise paulomycins $A_2$, D and E can be reacted under essentially the same conditions to give the corresponding $A_2$, D and B analogs.

Example 12 Reaction of Paulomycin A, $A_2$, B, D, and E with a 1-Deoxy-1-Thiopentitol or with a 1-Deoxy-1-thiohexitol

The 1-deoxy-1-thio-pentitol or -hexitol (20 eq) is dissolved in pH 7.85 phosphate buffer. The pH is then adjusted to 8.7 with 1N KOH solution and 1 equivalent of paulomycin (A, $A_2$, B, D or E) is added under stirring. After 1 hour at room temperature the solution is adjusted to pH 5.5 and passed over Amberlite XAD-4. The paulomycin-thio-pentitol or -hexitol reaction products are absorbed on the resin and eluted with acetone. The acetone solution is concentrated to dryness. The residue is dissolved in acetone or methylene chloride and this solution is mixed with ether-hexane mixture. The precipitated paulomycin-thio-pentitol or paulomycin-thiohexitol product is isolated by filtration and dried. Characterization of these materials is obtained by fast atom bombardment mass spectroscopy.

Example 12a Bis adduct of Paulomycin B with 1-Thiohexitol

1-Thiohexitol (1-deoxy-1-thio-D-glucitol, 5.86 g, which is commercially available or can be made by method well known in the art) is dissolved in potassium phosphate buffer solution (0.05M, pH 7.85, 230 ml) and this solution is adjusted to pH 8.7 by the addition of aqueous sodium hydroxide (N). To this rapidly stirred solution is added paulomycin B (2.0 g, 1 equiv); the solid dissolves rapidly to give a colourless solution.

After 18 hrs, the reaction solution is adjusted to pH 5.5 by the addition of aqueous hydrochloric acid (2N). This solution is applied to the top of a column of HP-20 resin (200 ml), and the column is washed with water until all of the thiosorbitol (and phosphate buffer) has been eluted. Elution of the column with acetonitrile-water (70:30) removed the adduct. The eluates are assayed by HPLC using a reversed phase C-18 analytical column; pools of those fractions containing desired product are made, volatile solvent is removed on a rotating evaporator at 30° in vacuo, and the aqueous residue is shell-frozen and lyophilised, yielding 3.16 g of colourless amorphous bis-adduct.

The product is characterized by FAB-MS(M·m/z 1168; $HOCH_2(CHOH)_4S$·m/z 197). It is highly soluble in water and is equal in antibacterial activity to $273a_1$ both in vitro and in vivo.

Results of the reactions of paulomycins A, $A_2$, B, D and E with N-acetyl-L-cysteine, the esters of N-acetyl-L-cysteine and the mercapto compounds of Chart II are presented in Table 1.

TABLE 1

PRODUCTS OF REACTION OF PAULOMYCIN A AND B WITH N-ACETYL-L-CYSTEINE ESTERS

| Paulomycin | Reactant | Compound | Molecular Formula* | Molecular Weight |
|---|---|---|---|---|
| A | N-acetyl-L-cysteine | 3a | $C_{44}H_{64}N_{4}O_{23}S_{3}$ | 1112* |
| $A_2$ | | 1x | $C_{44}H_{64}N_{4}O_{23}S_{3}$ | 1112 |
| B | | 1b (273 a/b) | $C_{43}H_{62}N_{4}O_{23}S_{3}$ | 1098* |
| D | | 1d | $C_{41}H_{58}N_{4}O_{23}S_{3}$ | 1070 |
| E | | 1e | $C_{39}H_{54}N_{4}O_{22}S_{3}$ | 1026 |
| A | N-acetyl-L-cysteine methyl ester | 2a | $C_{46}H_{68}N_{4}O_{23}S_{3}$ | 1140* |
| $A_2$ | | 2x | $C_{46}H_{68}N_{4}O_{23}S_{3}$ | 1140 |
| B | | 2b | $C_{45}H_{66}N_{4}O_{23}S_{3}$ | 1126* |
| D | | 2d | $C_{43}H_{62}N_{4}O_{23}S_{3}$ | 1098 |
| E | | 2e | $C_{41}H_{58}N_{4}O_{22}S_{3}$ | 1054 |
| A | N-acetyl-L-cysteine ethyl ester | 3a | $C_{48}H_{72}N_{4}O_{23}S_{3}$ | 1168* |
| $A_2$ | | 3x | $C_{46}H_{72}N_{4}O_{23}S_{3}$ | 1168 |
| B | | 3b | $C_{47}H_{70}N_{4}O_{23}S_{3}$ | 1154* |
| D | | 3d | $C_{45}H_{66}N_{4}O_{23}S_{3}$ | 1126 |
| E | | 3e | $C_{43}H_{62}N_{4}O_{22}S_{3}$ | 1082 |
| A | N-acetyl-L-cysteine butyl ester | 4a | $C_{52}H_{80}N_{4}O_{23}S_{3}$ | 1224* |
| $A_2$ | | 4x | $C_{52}H_{80}N_{4}O_{23}S_{3}$ | 1224 |
| B | | 4b | $C_{51}H_{78}N_{4}O_{23}S_{3}$ | 1210 |
| D | | 4d | $C_{49}H_{74}N_{4}O_{23}S_{3}$ | 1182 |
| E | | 4e | $C_{47}H_{70}N_{4}O_{22}S_{3}$ | 1138 |
| A | N-acetyl-L-cysteine octyl ester | 5a | $C_{60}H_{96}N_{4}O_{23}S_{3}$ | 1336* |
| $A_2$ | | 5x | $C_{60}H_{96}N_{4}O_{23}S_{3}$ | 1336 |
| B | | 5b | $C_{59}H_{94}N_{4}O_{23}S_{3}$ | 1322* |
| D | | 5d | $C_{57}H_{90}N_{4}O_{23}S_{3}$ | 1294 |
| E | | 5e | $C_{55}H_{86}N_{4}O_{22}S_{3}$ | 1250 |
| A | Mercaptoacetic acid | 6a | $C_{38}H_{54}N_{2}O_{21}S_{3}$ | 970* |
| $A_2$ | | 6x | $C_{38}H_{54}N_{2}O_{21}S_{3}$ | 970 |
| B | | 6b | $C_{37}H_{52}N_{2}O_{21}S_{3}$ | 956* |
| D | | 6d | $C_{35}H_{48}N_{2}O_{21}S_{3}$ | 928 |
| E | | 6e | $C_{33}H_{44}N_{2}O_{20}S_{3}$ | 884 |
| A | Mercaptopropionic acid | 7a | $C_{40}H_{58}N_{2}O_{21}S_{3}$ | 998* |
| $A_2$ | | 7x | $C_{40}H_{54}N_{2}O_{21}S_{3}$ | 998 |
| B | | 7b | $C_{39}H_{52}N_{2}O_{21}S_{3}$ | 984* |
| D | | 7d | $C_{37}H_{48}N_{2}O_{21}S_{3}$ | 956 |
| E | | 7e | $C_{35}H_{44}N_{2}O_{20}S_{3}$ | 912 |

* Determined by Fast Atom Bombardment Mass Spectroscopy. All others were calculated.

| Paulomycin | Reactant | Compound | Molecular Formula* | Molecular Weight |
|---|---|---|---|---|
| A | Thiomalic acid | 8a | $C_{42}H_{58}N_2O_{25}S_3$ | 1086* |
| $A_2$ | | 8x | $C_{42}H_{58}N_2O_{25}S_3$ | 1086 |
| B | | 8b | $C_{41}H_{56}N_2O_{25}S_3$ | 1072* |
| D | | 8d | $C_{39}H_{52}N_2O_{25}S_3$ | 1044 |
| E | | 8e | $C_{37}H_{48}N_2O_{24}S_3$ | 1000 |
| A | Cysteine | 9a | $C_{40}H_{60}N_4O_{21}S_3$ | 1028* |
| $A_2$ | | 9x | $C_{40}H_{60}N_4O_{21}S_3$ | 1028 |
| B | | 9b | $C_{39}H_{58}N_4O_{21}S_3$ | 1014* |
| D | | 9d | $C_{37}H_{54}N_4O_{21}S_3$ | 986 |
| E | | 9e | $C_{35}H_{50}N_4O_{20}S_3$ | 942 |
| A | Homocysteine | 10e | $C_{42}H_{64}N_4O_{21}S_3$ | 1056* |
| $A_2$ | | 10x | $C_{42}H_{64}N_4O_{21}S_3$ | 1056 |
| B | | 10b | $C_{41}H_{62}N_4O_{21}S_3$ | 1042* |
| D | | 10d | $C_{39}H_{58}N_4O_{21}S_3$ | 1014 |
| E | | 10e | $C_{37}H_{54}N_4O_{20}S_3$ | 970 |
| A | Glutathione | 11a | $C_{54}H_{80}N_8O_{29}S_3$ | 1400* |
| $A_2$ | | 11x | $C_{54}H_{80}N_8O_{29}S_3$ | 1400 |
| B | | 11b | $C_{54}H_{78}N_8O_{28}S_3$ | 1386* |
| D | | 11d | $C_{51}H_{74}N_8O_{29}S_3$ | 1358 |
| E | | 11e | $C_{49}H_{70}N_8O_{28}S_3$ | 1314 |
| A | Thioglucose | 12a | $C_{46}H_{70}N_2O_{27}S_3$ | 1178* |
| $A_2$ | | 12x | $C_{46}H_{70}N_2O_{27}S_3$ | 1178 |
| B | | 12b | $C_{45}H_{68}N_2O_{27}S_3$ | 1164* |
| D | | 12d | $C_{43}H_{64}N_2O_{27}S_3$ | 1136 |
| E | | 12e | $C_{41}H_{60}N_2O_{26}S_3$ | 1092 |
| A | Thioglycerol | 13a | $C_{40}H_{60}N_2O_{21}S_3$ | 1002* |
| $A_2$ | | 13x | $C_{40}H_{60}N_2O_{21}S_3$ | 1002 |
| B | | 13b | $C_{39}H_{60}N_2O_{21}S_3$ | 988* |
| D | | 13d | $C_{37}H_{56}N_2O_{21}S_3$ | 960 |
| E | | 13e | $C_{35}H_{52}N_2O_{20}S_3$ | 916 |
| A | 1-deoxy-1-thio-pentitol | 14a | $C_{44}H_{70}N_2O_{25}S_3$ | 1122* |
| $A_2$ | | 14x | $C_{44}H_{70}N_2O_{25}S_3$ | 1122 |
| B | | 14b | $C_{44}H_{68}N_2O_{25}S_3$ | 1108* |
| D | | 14d | $C_{41}H_{64}N_2O_{25}S_3$ | 1080 |
| E | | 14e | $C_{39}H_{60}N_2O_{24}S_3$ | 1036 |
| A | 1-deoxy-1-thio hexitol | 15a | $C_{46}H_{74}N_2O_{27}S_3$ | 1182* |
| $A_2$ | | 15x | $C_{46}H_{74}N_2O_{27}S_3$ | 1182 |
| B | | 15b | $C_{45}H_{72}N_2O_{27}S_3$ | 1168* |
| D | | 15d | $C_{43}H_{68}N_2O_{27}S_3$ | 1140 |
| E | | 15e | $C_{41}H_{64}N_2O_{26}S_3$ | 1096 |

* Determined by Fast Atom Bombardment Mass Spectroscopy. All others were calculated.

CHARACTERIZATION OF ANTIBIOTICS $273a_1$

ANTIBIOTIC $273a_{1}\alpha$ (Prep. -145.1)

1. Appearance: Colorless amorphous acidic material

2. Solubility: Soluble in lower alcohols, ketones, ethyl acetate; less soluble in chloroform, methylene chloride; insoluble in ether and saturated hydrocarbon solvents. The free acid form is insoluble in water, but soluble in phosphate buffers at physiological pH (7.0-7.5). Salts are soluble in water.

3. Molecular Formula: $C_{44}H_{64}N_{4}O_{23}S_{3}$

4. Molecular Weight: Calcd: 1112. Found: 1112 (by FAB-MS).

5. Anal. Calcd/Found: C = 47.48/46.82; H = 5.75/5.78; N = 5.03/4.93; S = 8.63/8.72.

6. Ash: 0.04%

7. $[\alpha]_D^{25}$: -31° (C, 0.905, MeOH)

8. Melting Point: 120° (decomposition)

9. Potentiometric Titration: Solvent, 60% aqueous ethanol; titrant, KOH; eq. weight, First break (weak), 577; second break, 379.

10. IR Spectrum: Tabulation of the IR bands in NUJOL mull follows.

11. UV Spectrum: The UV spectrum of antibiotic $273a_1$ in methanol is as follows:

| λ max | a |
|---|---|
| 248 | 16.20 |
| 274 | 8.51 |
| 321 | 8.09 |

ANTIBIOTIC $273a_{1}\beta$ (Prep. -147.1)

1. Appearance: Colorless amorphous acidic material.

2. Solubility: Soluble in lower alcohols, ketones, ethyl acetate, less soluble in chloroform, methylene chloride; insoluble in ether and saturated hydrocarbon solvents. The free acid form is insoluble in water but soluble in phosphate buffer at physiological pH's (7.0-7.5). Salts are soluble in water.

3. Molecular Formula: $C_{43}H_{62}N_{4}O_{23}S_{3}$

4. Molecular Weight: Calcd = 1098; found = 1098 (FAB-MS).

5. Anal. Calcd/Found: C,46.99/46.22; H, 5.64/5.69; N, 5.10/5.00; S, 8.74/8.83.

6. Ash: 0.17%

7. $[\alpha]_D^{25}$: -35° (C, 0.908, methanol).

8. Melting Point: 120° (decomposition).

9. Potentiometric Titration: Solvent: 60% aqueous ethanol; titrant: KOH; equivalent weight: first break, 640; second break, 399.

10. IR Spectrum: Tabulation of the IR bands in NUJOL mull follows.

11. UV Spectrum: The UV spectrum of antibiotic 273a$_{1}\beta$ in methanol is as follows:

| λ max | a |
|---|---|
| 248 | 16.29 |
| 274 | 8.56 |
| 321 | 8.21 |

ANTIBIOTIC 273a$_1$ (Prep. -152.1)

Prep. -152.1 was obtained by reaction of paulomycin (a mixture of 60% paulomycin A and 40% of paulomycin B) with N-acetyl-L-cysteine. Antibiotic 273a$_1$ obtained from this reaction was found to be a mixture of 40% antibiotic 273a$_{1}\alpha$ and 40% of 273a$_{1}\beta$.

1. Appearance: Colorless amorphous acidic material.

2. Solubility: Soluble in lower alcohols, ketones, ethyl acetate; less soluble in chloroform, methylene chloride; insoluble in ether and saturated hydrocarbon solvents. The free acid form is insoluble in water but soluble in phosphate buffer at physiological pH's (7.0-7.5). Salts are soluble in water.

3. Molecular Formula: Mixture of $C_{44}H_{64}N_4O_{23}S_3$ (60%) and $C_{43}H_{62}N_4O_{23}S_3$ (40%).

4. Molecular Weight: Calcd = 1112, 1098. Found = 1112, 1098.

5. Anal. Calcd/Found: C, 47.24/46.42; H, 5.70/5.68; N, 5.06/4.90; S, 8.68/8.73.

6. Ash: 0.16%

7. $[\alpha]_D^{25}$: -33° (C, 0.890, methanol).

8. Melting Point: 120° (decomposition).

9. Potentiometric Titration: Solvent, 60% ethanol; titrant, KOH; equivalent weight, first break, 576; second break, 376.

10. IR Spectrum: Tabulation of the IR bands in NUJOL mull follows.

11. U.V. Spectrum: The UV spectrum of antibiotic 273a$_1$ in methanol is as follows:

| λ max | a |
|---|---|
| 248 | 16.45 |
| 274 | 8.61 |
| 322 | 8.31 |

IR BAND TABULATION OF ANTIBIOTIC 273a$_{1\alpha}$

| Band Frequency | Intensity | Type | Band Frequency | Intensity | Type |
|---|---|---|---|---|---|
| 3469.9 | 55 | SH | 1735.9 | 4 | AVG |
| 3351.3 | 34 | BRD | 1661.6 | 19 | AVG |
| 3272.2 | 34 | BRD | 1626.9 | 21 | AVG |

| Band Frequency | Intensity | Type | Band Frequency | Intensity | Type |
|---|---|---|---|---|---|
| 3238.4 | 36 | SH | ------ | -- | --- |
| 2952.0 | 0 | BRD M | 1574.8 | 23 | AVG |
| 2913.4 | 0 | BRD M | 1532.4 | 22 | BRD |
| 2868.1 | 3 | SH M | 1460.1 | 7 | AVG M |
| 2854.6 | 1 | AVG M | 1377.1 | 7 | AVG M |
| 2724.4 | 65 | BRD M | 1343.4 | 25 | SH |
| 1949.0 | 88 | BRD | 1299.0 | 18 | AVG |

| Band Frequency | Intensity | Type | Band Frequency | Intensity | Type |
|---|---|---|---|---|---|
| 1261.4 | 21 | SH | 932.5 | 59 | AVG |
| 1243.1 | 17 | AVG | 911.3 | 49 | AVG |
| 1190.0 | 23 | AVG | 894.0 | 54 | AVG |
| 1120.6 | 20 | AVG | 872.7 | 62 | SH |
| 1098.4 | 25 | AVG | 833.2 | 61 | AVG |
| 1056.0 | 29 | AVG | 816.8 | 56 | SHP |
| 1026.1 | 25 | AVG | 768.6 | 64 | SH |
| 994.3 | 28 | AVG | 722.3 | 53 | AVG M |
| 975.0 | 44 | SH | 689.5 | 52 | AVG |

Band Frequency: Band frequencies in wavenumbers ($CM^{-1}$)

Intensity: Intensity in percent transmittance (%T)

Data Type in Local Peak Region: BRD = Broad; AVG = Average; SHP = Sharp; SH = Shoulder.

This peak list is unedited.

M: Possible interference from mineral oil.

25 STRONGEST PEAKS

| %T | Frequency |
|---|---|
| 0 | 2952.0 |
| 0 | 2913.3 |
| 1 | 2854.5 |
| 3 | 2868.0 |
| 4 | 1735.8 |
| 7 | 1460.0 |
| 7 | 1377.0 |
| 17 | 1243.0 |
| 18 | 1299.0 |
| 19 | 1661.5 |
| 20 | 1120.5 |
| 21 | 1626.8 |
| 21 | 1627.8 |
| 21 | 1261.3 |
| 22 | 1532.3 |
| 23 | 1574.7 |
| 23 | 1190.0 |
| 25 | 1343.3 |
| 25 | 1098.3 |
| 25 | 1026.0 |
| 28 | 994.2 |
| 29 | 1056.0 |
| 34 | 3351.2 |
| 34 | 3272.1 |
| 36 | 3238.3 |

Prep.: Mineral Oil Mull

Max %T: 95 #3746.7

%T at 3800 ($CM^{-1}$): 95

Density ($CM^{-1}$/PT): 0.964

IR BAND TABULATION OF ANTIBIOTIC $273a_{1\beta}$

| Band Frequency | Intensity | Type | Band Frequency | Intensity | Type |
|---|---|---|---|---|---|
| 3466.0 | 60 | SH | 1735.9 | 7 | AVG |
| 3350.3 | 40 | BRD | 1661.6 | 24 | AVG |
| 3273.1 | 40 | BRD | 1627.9 | 27 | AVG |
| 3237.5 | 42 | SH | 1574.8 | 29 | AVG |
| 2953.9 | 0 | BRD M | 1530.5 | 27 | BRD |
| 2915.3 | 0 | BRD M | 1458.1 | 10 | AVG M |
| 2868.1 | 4 | SH M | 1377.1 | 11 | AVG M |
| 2854.6 | 2 | AVG M | 1345.3 | 30 | SH |
| 2725.4 | 70 | BRD M | 1300.0 | 23 | AVG |
| 1925.9 | 91 | BRD | 1245.0 | 24 | AVG |

| Band Frequency | Intensity | Type | Band Frequency | Intensity | Type |
|---|---|---|---|---|---|
| 1227.6 | 25 | AVG | 929.6 | 62 | AVG |
| 1202.6 | 29 | BRD | 910.3 | 56 | AVG |
| 1156.3 | 33 | AVG | 893.0 | 60 | AVG |
| 1122.5 | 26 | BRD | 854.4 | 67 | AVG |
| 1099.4 | 31 | AVG | 836.1 | 66 | AVG |
| 1064.7 | 37 | SH | 816.8 | 63 | SHP |
| 1055.0 | 36 | AVG | 767.6 | 68 | SH |
| 1026.1 | 32 | AVG | 722.3 | 57 | AVG M |
| 995.2 | 34 | AVG | 689.5 | 57 | AVG |
| 973.0 | 52 | SH | | | |

Band Frequency: Band frequencies in wavenumbers ($CM^{-1}$)

Intensity: Intensity in percent transmittance (%T)

Data Type in Local Peak Region: BRD = broad; AVG = average; SHP = sharp; SH = shoulder.

This peak list is unedited.

M: Possible interference from mineral oil.

25 STRONGEST PEAKS

| %T | Frequency |
|---|---|
| 0 | 2953.8 |
| 0 | 2915.2 |

| %T | Frequency |
|---|---|
| 2 | 2854.5 |
| 4 | 2868.0 |
| 7 | 1735.8 |
| 10 | 1458.0 |
| 11 | 1377.0 |
| 23 | 1300.0 |
| 24 | 1661.5 |
| 24 | 1245.0 |
| 25 | 1227.5 |
| 26 | 1122.5 |
| 27 | 1627.8 |
| 27 | 1530.5 |
| 29 | 1574.7 |
| 29 | 1202.5 |
| 30 | 1345.2 |
| 31 | 1099.3 |
| 32 | 1026.0 |
| 33 | 1156.2 |
| 34 | 995.1 |
| 36 | 1055.0 |
| 37 | 1064.6 |
| 40 | 3350.2 |
| 40 | 3273.0 |

Prep.: Mineral oil mull

Max %T: 97 #3772.7

%T at 3800 ($CM^{-1}$): 96

Density ($CM^{-1}$/Pt: 0.964

## IR BAND TABULATION OF ANTIBIOTIC 273a$_1$

| Band Frequency | Intensity | Type | Band Frequency | Intensity | Type |
|---|---|---|---|---|---|
| 3476.6 | 49 | SH | 1946.1 | 87 | BRD |
| 3348.4 | 26 | BRD | 1736.8 | 1 | AVG |
| 3272.2 | 25 | BRD | 1662.6 | 11 | AVG |
| 3239.4 | 27 | SH | 1626.9 | 14 | AVG |
| 2958.7 | 0 | BRD M | 1575.8 | 15 | AVG |
| 2867.1 | 2 | SH M | 1532.4 | 14 | BRD |
| 2853.6 | 1 | AVG M | 1457.2 | 3 | AVG M |
| 2728.3 | 60 | BRD M | 1377.1 | 4 | AVG M |
| 2675.2 | 63 | SH M | 1344.3 | 17 | SH |
| 2631.8 | 64 | SH | 1299.0 | 11 | AVG |
| 2537.3 | 69 | SH | 1260.4 | 14 | SH |

| Band Frequency | Intensity | Type | Band Frequency | Intensity | Type |
|---|---|---|---|---|---|
| 1244.0 | 11 | AVG | 911.3 | 42 | AVG |
| 1230.5 | 12 | SH | 893.0 | 47 | AVG |
| 1191.0 | 16 | AVG | 868.9 | 57 | AVG |
| 1120.6 | 13 | AVG | 855.4 | 57 | BRD |
| 1098.4 | 17 | AVG | 834.2 | 56 | AVG |

| Band Frequency | Intensity | Type | Band Frequency | Intensity | Type |
|---|---|---|---|---|---|
| 1055.0 | 21 | AVG | 816.8 | 50 | SHP |
| 1026.1 | 18 | AVG | 768.6 | 59 | SH |
| 995.2 | 20 | AVG | 722.3 | 47 | AVG M |
| 973.0 | 37 | SH | 689.5 | 46 | AVG |
| 930.6 | 51 | AVG | 651.9 | 41 | BRD |

Band Frequency: Band frequencies in wavenumbers ($CM^{-1}$).

Intensity: Intensity in percent transmittance (%T).

Data Type in Local Peak Region: BRD = broad; AVG = average; SHP = sharp; SH = shoulder.

This peak list is unedited.

M: Possible interference from mineral oil.

25 STRONGEST PEAKS

| | |
|---|---|
| 0 | 2958.6 |
| 1 | 2853.5 |
| 1 | 1736.7 |
| 2 | 2867.0 |
| 3 | 1457.1 |
| 4 | 1377.0 |
| 11 | 1662.5 |
| 11 | 1299.0 |
| 11 | 1244.0 |
| 12 | 1230.5 |
| 13 | 1120.5 |
| 14 | 1626.8 |
| 14 | 1532.3 |
| 14 | 1260.3 |
| 15 | 1575.7 |
| 16 | 1191.0 |
| 17 | 1344.2 |
| 17 | 1098.3 |
| 18 | 1026.0 |
| 20 | 995.1 |
| 21 | 1055.0 |
| 25 | 3272.1 |
| 26 | 3348.3 |
| 27 | 3239.3 |
| 37 | 973.0 |

Prep.: Mineral oil mull.
Max %T: 98 #3772.7
%T at 3800 ($CM^{-1}$): 97
Density ($CM^{-1}$/Pt): 0.964.

Antibiotics $273a_1$, and certain of the adducts and derivatives prepared therefrom, can form salts with divalent cations like Ca (calcium) or Mg (magnesium) and trivalent cations like Al (aluminum). Salts can also be formed with quarternary ammonium cations.

The salts of antibiotic $273a_1$, $273a_1\alpha$ and $273a_1\beta$ and their derivatives are especially desirable. Antibiotics $273a_1$, $273a_1\alpha$ and $273a_1\beta$ are tribasic acids with two carboxyl groups of approximate pKa values of 3.7, 4.0 and an enolic hydroxyl, pKa about 6.5 (in water). As tribasic acidic compounds, antibiotics $273a_1$ can form mono-, di- or tri-cation salts with inorganic monovalent cations like sodium, potassium, and lithium as well as organic amines like methyl, ethyl and in general straight chain, branched or cyclic amines; unsaturated straight chain or branched or cyclic alkyl amines; aromatic or heterocyclic aryl amines, ammonium, or combinations of all the above. The amines can be primary, secondary, or tertiary.

Especially preferred metal cations are those derived from the alkali metals, e.g. sodium, and potassium, from the alkaline earth metals, e.g. calcium, barium, transition metals, e.g. aluminum, zinc, and iron.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, and the like, aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereof, e.g., 1-methylpiperidine, 4-ethylmorpholine, 1-isopropylpyrrolidine, 2-methylpyrrolidine, 1,4-dimethylpiperazine, 2-methylpiperidine, and the like, as well as amines containing water-solubilizing or hydrophilic groups, e.g., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl)aminomethane, N-phenylethanolamine, N-(p-tert-amylphenyl)-diethanolamine, galactamine, N-methylglucosamine, ephedrine, phenylephrine, epinephrine, procaine, and the like. Further useful amine salts are the basic amino acid salts, e.g , lysine and arginine.

Salt formation occurs by replacement of the hydrogen of the 2-hydroxy group by a pharmacologically acceptable cation as well as by replacement of hydrogen in the carboxyl moieties.

Examples of suitable pharmacologically acceptable quarternary ammonium cations are tetramethylammonium, tetraethylammonium, benzyltrimethylammonium, phenyltriethylammonium, and the like.

Salts of antibiotics $273a_1$, $273A_1\alpha$ and $273a_1\beta$ are prepared by methods known in the art. For example, by reacting a solution of the antibiotic with aqueous organic or inorganic base. The mono-, di- or tri-cation salt will be obtained dependent upon whether one, two or three moles of the base is used.

## SALTS

Example 13 Antibiotic $273a_1$ Disodium Salt

Antibiotic $273a_1$ (mixture of antibiotics $273a_1\alpha$ and $273a_1\beta$; 1105 mg (1 mmole) was dissolved in 12 ml of t-butanol. Water, 11 ml, and 2 ml of 1N aqueous sodium hydroxide were added. The resulting solution which had a pH of 6.2 was freeze-dried to give preparation -55.2, 1.18g. Characterization of this material follows.

## Characterization of Antibiotic 273α1 Disodium Salt

1. Appearance: Colorless amorphous material.
2. Solubility: Soluble in water and aqueous buffer systems, lower alcohols (methanol; ethanol). Insoluble in chloroform, methylene chloride and saturated hydrocarbon solvents.
3. Molecular Formula: 273a 1α disodium salt: $C_{44}H_{62}N_4O_{23}S_3Na_2$
   273a 1β disodium salt: $C_{43}H_{60}N_4O_{23}S_3Na_2$
4. Molecular Weight: Calcd for $C_{44}H_{62}N_4O_{23}S_3Na_2$: 1156
   Calcd for $C_{43}H_{60}N_4O_{23}S_3Na_2$: 1142
   Found for prep. 17411-ADA-55.2: 1142 and 1156 (Fast atom bombardment mass spectrum).
5. Anal. calcd for 56.3% of 273a 1α and 43.7% of 273a 1β: C, 45.45; H, 5.31; N, 4.86; S, 8.35; Na, 4.00. Corrected for ca. 4% water and 5% t-butanol; C, 44.60; H, 5.34; N, 4.42; S, 7.60; Na, 3.64. Found: C, 43.23; H, 5.44; N, 4.18; S, 7.39; Na, 4.04.
6. Weight Loss at 60°: 3.27%
7. Water (KF): 3.94%
8. Ash at 700° (Na): 4.04%
9. Melt Solvant: Some decomposition; t-butanol, 4.8%
10. $[\alpha]_D^{25}$,-8° (0.845, methanol)
11. Melting Point: ca. 170° (decomposition)
12. Potentiometric Titration: Solvent: 60% aqueous ethanol
    Titrant: HCl
    Eq. Weight: 650
13. HPLC Analysis: Results indicate the presence of 56.3% of 273a 1α disodium salt and 43.7% of 273a 1β disodium salt.
14. IR Spectrum:

BAND TABULATION OF THE INFRARED SPECTRUM 273a1 DISODIUM SALT

| Band Frequency | Intensity | Type | Band Frequency | Intensity | Type |
|---|---|---|---|---|---|
| 3411.6 | 47 | SH | 1133.1 | 28 | SH |
| 3359.0 | 32 | BRD | 1119.6 | 26 | AVG |
| 2953.0 | 0 | BRD M | 1097.4 | 32 | AVG |
| 2914.4 | 0 | BRD M | 1056.0 | 33 | AVG |
| 2868.1 | 2 | SH M | 1027.0 | 30 | AVG |
| 2854.6 | 1 | AVG M | 995.2 | 31 | AVG |
| 2727.3 | 73 | BRD M | 975.0 | 49 | SH |
| 2679.1 | 77 | SH M | 928.7 | 62 | SH |
| 1734.9 | 15 | AVG | 911.3 | 52 | AVG |
| 1648.1 | 18 | SH | 894.9 | 59 | AVG |
| 1601.8 | 8 | AVG | 854.4 | 61 | AVG |
| 1542.0 | 26 | BRD | 839.0 | 64 | SH |
| 1461.0 | 7 | AVG M | 815.8 | 60 | AVG |
| 1377.1 | 7 | AVG M | 780.2 | 64 | SH |
| 1343.4 | 30 | SH | 746.4 | 55 | SH |
| 1298.0 | 23 | AVG | 722.3 | 48 | AVG M |
| 1263.3 | 28 | AVG | 687.6 | 50 | SH |
| 1245.0 | 27 | AVG | 668.3 | 47 | BRD |
| 1192.9 | 33 | AVG | | | |
| 1151.5 | 33 | SH | | | |

Band Freq.: Band frequencies in wavenumbers ($CM^{-1}$)

Inten.: Intensity in percent transmittance (%T)

Data type in local peak region: BRD - Broad AVE - Average SHP - Sharp SH - Shoulder

This peak list is unedited

M: Possible interference from mineral oil

25 STRONGEST PEAKS

| %T | Frequency |
|---|---|
| 0 | 2953.0 |
| 0 | 2914.3 |
| 1 | 2854.5 |
| 2 | 2868.0 |

## 25 STRONGEST PEAKS

| %T | Frequency |
|---|---|
| 7 | 1461.0 |
| 7 | 1377.0 |
| 8 | 1601.7 |
| 15 | 1734.8 |
| 18 | 1648.0 |
| 23 | 1298.0 |
| 26 | 1542.0 |
| 26 | 1119.5 |
| 27 | 1245.0 |
| 28 | 1263.2 |
| 28 | 1133.0 |
| 30 | 1343.3 |
| 30 | 1027.0 |
| 31 | 995.1 |
| 32 | 3359.0 |
| 32 | 1097.3 |
| 33 | 1192.8 |
| 33 | 1151.5 |
| 33 | 1056.0 |
| 47 | 3477.5 |
| 47 | 668.2 |

Prep.: Mineral oil mull.
Max %T: 93 #3765.0
%T at 3800 ($CM^{-1}$): 92
Density ($CM^{-1}$/Pt): 0.964

15. UV Spectrum:

| λmax | a |
|---|---|
| 249 | 14.83 |
| 275 | 8.66 |
| 322 | 7.21 |

Example 14 Antibiotic $273a_1$ Trisodium Salt

Antibiotic $273a_1$ free acid (1.3537 g; 1.255 mmoles) was dissolved in 50 ml of t-butanol and 25 ml of water. Aqueous sodium hydroxide (0.5 N; 7.35 ml; 3.765 mmoles) was added with simultaneous determination of the pH of the solution. The titration curve is shown in Figure 1. The solution was then freeze-dried for 96 hours to give prep. 18025-FBS-76.1 (18118-ADA-58.1),1.22 g.

Anal. Calcd for $C_{44}H_{61}N_4O_{23}Na_3$; C, 44.46; H, 5.36; N, 4.77; S, 8.18. Found: C, 42.15; H, 5.13; N, 4.41; S, 7.56. Corrected for 6.49% water content: C, 45.08; H, 4.72; S, 7.70; Sodium ash, 5.95%; calculated sodium for trisodium salt, 5.84:. Weight loss, 0.98%. Water (KF), 6.49%. Melt Solvate, decomposition bands $[\alpha]_D25$, + 15° (C, 0.89, water).

The IR Spectrum and IR Absorption bands follow:

UV spectrum (in water): λmax(ε)

251 nm (16550)
271 sh (9500)
332 (7850)

The HPLC chromatographic analysis of the trisodium salt of $273a_1$ (58.1) indicated the absence of $273a_1$-mycinones. Antibiotic $273a_1$ \9273$a_{1\alpha}$ and $273a_{1\beta}$) accounted for about 97.26% of the materials present in prep 58.1. The HPLC Chromatograms of $273a_1$-trisodium salt obtained immediately after preparation and 21 and 35 days later are presented in Figures 3, 4 and 5, respectively, Conditions used for the HPLC chromatograms are noted in Tables 2, 3 and 4. The Fast Atom Bombardment mass spectrum (positive ions) of $273a_1$, trisodium salt is presented in Figure 6.

BAND TABULATION OF THE INFRARED SPECTRUM

OF ANTIBIOTIC 273a$_1$ TRISODIUM SALT

| BAND FREQ. | INTEN. | TYPE | | BAND FREQ. | INTEN. | TYPE | |
|---|---|---|---|---|---|---|---|
| 3488.2 | 52 | SH | | 1642.3 | 27 | SH | |
| 3371.5 | 40 | BRD | | 1594.1 | 11 | AVG | |
| 2953.9 | 2 | AVG | M | 1542.0 | 42 | SH | |
| 2925.0 | 0 | BRD | M | 1461.0 | 22 | AVG | M |
| 2868.1 | 9 | SH | M | 1377.1 | 18 | AVG | M |
| 2854.6 | 5 | AVG | M | 1301.9 | 36 | AVG | |
| 2727.3 | 82 | BRD | M | 1270.1 | 41 | SH | |
| 1732.0 | 34 | AVG | | 1249.8 | 44 | SH | |
| 1192.9 | 50 | AVG | | 933.5 | 73 | AVG | |
| 1152.4 | 49 | AVG | | 911.3 | 69 | AVG | |
| 1118.7 | 42 | AVG | | 894.0 | 71 | AVG | |
| 1097.4 | 48 | AVG | | 864.1 | 67 | AVG | |
| 1055.0 | 46 | AVG | | 830.3 | 66 | AVG | |
| 1027.0 | 46 | AVG | | 740.6 | 60 | SH | |
| 994.3 | 47 | AVG | | 721.3 | 55 | AVG | M |
| 974.0 | 62 | SH | | | | | |

BAND FREQ.: BAND FREQUENCIES IN WAVENUMBERS (CM-1)
INTEN.: INTENSITY IN PERCENT TRANSMITTANCE (%T)
DATA TYPE IN LOCAL PEAK REGION: BRD - BROAD; AVG - AVERAGE; SHP - SHARP; SH - SHOULDER. THIS PEAK LIST IS UNEDITED.
M: POSSIBLE INTERFERENCE FROM MINERAL OIL

25 STRONGEST PEAKS

| %T | FREQ. | %T | FREQ. |
|---|---|---|---|
| 0 | 2925.0 | 42 | 1118.6 |
| 2 | 2953.8 | 44 | 1249.7 |
| 5 | 2854.5 | 46 | 1055.0 |
| 9 | 2868.0 | 46 | 1027.0 |
| 11 | 1594.0 | 47 | 994.2 |
| 18 | 1377.0 | 48 | 1097.3 |
| 22 | 1461.0 | 49 | 1152.3 |
| 27 | 1642.2 | 50 | 1192.8 |
| 34 | 1732.0 | 52 | 3488.1 |
| 36 | 1301.8 | 55 | 721.2 |
| 40 | 3371.5 | 60 | 740.5 |
| 41 | 1270.0 | 62 | 974.0 |
| 42 | 1542.0 | | |

PREP: MINERAL OIL MULL
TAPE: 129 FILE : 39
MAX %T: 95 #1920.1
%T AT 3800 (CM-1): 91
DENSITY (CM-1/PT): 0.964

Example 15 Antibiotic $273a_1$ Tripotassium Salt

Antibiotic $273a_1$ free acid (1.2813 g; 1.157 mmoles) was dissolved in 50 ml of t-butanol and 25 ml of water. Aqueous potassium hydroxide (0.5N; 6.94 ml; 3.471 mmoles) was added with simultaneous determination of the pH of the solution. The filtration curve is shown in Figure 7. The solution was then freeze-dried for 96 hours to give preparation FBS-96.1, 1.2 g. Anal. calcd for $C_{44}H_{61}N_4O_{23}S_3K_3$: C, 42.96; H, 5.21; N, 4.55; S, 7.81; Found:C, 41.42; H, 5.05; N, 4.36; S, 6.90. Corrected for water content: C, 43.86; H, 5.30; N, 4.61; S, 7.31. Potassium ash, 9.79; calculated potassium for tripotassium salt, 9.52; corrected for water content 9,92. Water (KF), 5.90. Melt solvate, no solvents present. $[\alpha]_D^{25}$, + 13° (C, 1.016, water).

The IR spectrum and IR absorption bands follows.

BAND TABULATION OF THE INFRARED SPECTRUM

ANTIBIOTIC 273a$_1$ TRIPOTASSIUM SALT

| BAND FREQ. | INTEN. | TYPE | | BAND FREQ. | INTEN. | TYPE | |
|---|---|---|---|---|---|---|---|
| 3373.4 | 37 | BRD | | 1645.2 | 30 | AVG | |
| 2954.9 | 1 | AVG | M | 1594.1 | 9 | AVG | |
| 2924.0 | 0 | BRD | M | 1543.0 | 44 | SH | |
| 2868.1 | 7 | SH | M | 1461.0 | 21 | AVG | M |
| 2854.6 | 3 | AVG | M | 1411.8 | 46 | SH | |
| 2726.3 | 85 | BRD | M | 1378.1 | 17 | AVG | M |
| 2198.8 | 97 | BRD | | 1300.9 | 38 | AVG | |
| 1733.0 | 36 | AVG | | 1267.2 | 42 | AVG | |
| 1193.9 | 51 | AVG | | 933.5 | 77 | AVG | |
| 1152.4 | 51 | AVG | | 909.4 | 72 | AVG | |
| 1119.6 | 43 | AVG | | 895.9 | 73 | AVG | |
| 1095.5 | 50 | AVG | | 866.0 | 72 | AVG | |
| 1055.0 | 48 | AVG | | 831.3 | 72 | AVG | |
| 1027.0 | 47 | AVG | | 736.8 | 62 | SH | |
| 995.2 | 48 | AVG | | 721.3 | 57 | AVG | M |
| 974.0 | 66 | SH | | 668.3 | 57 | BRD | |

BAND FREQ.: BAND FREQUENCIES IN WAVENUMBERS (CM-1)
INTEN.: INTENSITY IN PERCENT RANSMITTANCE (%T)
DATA TYPE IN LOCAL PEAK REGION: BRD - BROAD; AVG - AVERAGE; SHP - SHARP; SH - SHOULDER. THIS PEAK LIST IS UNEDITED.
M: POSSIBLE INTERFERENCE FROM MINERAL OIL

25 STRONGEST PEAKS

| %T | FREQ. | %T | FREQ. |
|---|---|---|---|
| 0 | 2924.0 | 44 | 1543.0 |
| 1 | 2954.8 | 46 | 1411.7 |
| 3 | 2854.5 | 47 | 1027.0 |
| 7 | 2868.0 | 48 | 1055.0 |
| 9 | 1594.0 | 48 | 995.1 |
| 17 | 1378.0 | 50 | 1095.5 |
| 21 | 1461.0 | 51 | 1193.8 |
| 30 | 1645.1 | 51 | 1152.3 |
| 36 | 1733.0 | 57 | 721.2 |
| 37 | 3373.3 | 57 | 668.2 |
| 38 | 1300.8 | 62 | 736.7 |
| 42 | 1267.1 | 66 | 974.0 |
| 43 | 1119.5 | | |

PREP: MINERAL OIL MULL
TAPE: 129 FILE: 179
MAX %T: 99 #1960.6
%T AT 3800 (CM-1): 98
DENSITY (CM-1/PT): 0.964

UV spectrum (in water): λmax(ε)

251 nm (16650)

271 sh (9500)

333 (7850)

The HPLC chromatographic analysis of the tripotassium salt is presented in Figure 9. Conditions used for the HPLC chromatogram are noted in Table 6.

Example 16 Antibiotic 273a$_1$ Tris-(trihydroxyaminomethane) Salt

Antibiotic 273a$_1$ free acid (1.4905 g; 1.346 mmoles) was dissolved in 50 ml of t-butanol and 25 ml of water. Aqueous solution of trihydroxyaminomethane 0.5 M, 8.1 ml; 4.04 mmoles) was added with simultaneous determination of the pH of the solution. The titration curve is shown in Figure 10. The solution was then freeze-dried for 96 hours to give prep. 18025-FBS-96.1, 1.68 g. Anal. Calcd for $C_{44}H_{67}N_4O_{23}S_3$. $(C_4H_{11}O_3n_3)_3$: C, 45.46; H, 6.76; B, 6.63; S, 6.49. Found: C, 45.62; 7.32; N, 6.44; S, 5.87. Water (KF), 1.52; inorganic residue 0.22%; melt solvate, no solvent was identified, $[\alpha]_D^{25}$, + 9° (C, 0.699, water).

The IR spectrum and IR absorption bands follows:

UV spectrum (in water): λmax(ε)

251 nm (16700)

271 sh (9800)

333 (7700)

BAND TABULATION OF THE INFRARED SPECTRUM

OF ANTIBIOTIC 273a$_1$ TRIS SALT

| BAND FREQ. | INTEN. | TYPE | | BAND FREQ. | INTEN. | TYPE | |
|---|---|---|---|---|---|---|---|
| 3286.6 | 18 | BRD | | 1640.4 | 21 | SH | |
| 2953.0 | 2 | BRD | M | 1591.2 | 7 | AVG | |
| 2923.1 | 0 | BRD | M | 1530.5 | 22 | BRD | |
| 2870.0 | 7 | AVG | M | 1462.0 | 17 | AVG | M |
| 2854.6 | 4 | AVG | M | 1411.8 | 28 | SH | |
| 2732.1 | 52 | SH | M | 1378.1 | 12 | AVG | M |
| 2675.2 | 57 | SH | M | 1301.9 | 28 | AVG | |
| 2097.5 | 86 | BRD | | 1267.2 | 33 | BRD | |
| 1733.0 | 29 | AVG | | 1246.0 | 34 | AVG | |
| 1192.9 | 35 | AVG | | 910.3 | 57 | AVG | |
| 1150.5 | 40 | SH | | 895.9 | 60 | AVG | |
| 1119.6 | 33 | AVG | | 865.0 | 62 | AVG | |
| 1057.9 | 19 | AVG | | 830.3 | 64 | AVG | |
| 1027.0 | 26 | AVG | | 784.0 | 63 | SH | |
| 995.2 | 36 | AVG | | 748.3 | 52 | AVG | |
| 973.0 | 56 | SH | | 721.3 | 49 | AVG | M |
| 931.6 | 66 | SH | | 670.2 | 47 | BRD | |

BAND FREQ.: BAND FREQUENCIES IN WAVENUMBERS (CM-1)
INTEN.: INTENSITY IN PERCENT RANSMITTANCE (%T)
DATA TYPE IN LOCAL PEAK REGION: BRD - BROAD; AVG - AVERAGE; SHP - SHARP; SH - SHOULDER. THIS PEAK LIST IS UNEDITED.
M: POSSIBLE INTERFERENCE FROM MINERAL OIL

25 STRONGEST PEAKS

| %T | FREQ. | %T | FREQ. |
|---|---|---|---|
| 0 | 2923.0 | 28 | 1301.8 |
| 2 | 2953.0 | 29 | 1733.0 |
| 4 | 2854.5 | 33 | 1267.1 |
| 7 | 2870.0 | 33 | 1119.5 |
| 7 | 1591.1 | 34 | 1246.0 |
| 12 | 1378.0 | 35 | 1192.8 |
| 17 | 1462.0 | 36 | 995.1 |
| 18 | 3286.5 | 40 | 1150.5 |
| 19 | 1057.8 | 47 | 670.1 |
| 21 | 1640.3 | 49 | 721.2 |
| 22 | 1530.5 | 52 | 2732.0 |
| 26 | 1027.0 | 52 | 748.2 |
| 28 | 1411.7 | | |

PREP: MINERAL OIL MULL
TAPE: 129 FILE: 160
MAX %T: 92 #3743.8
%T AT 3800 (CM-1): 92
DENSITY (CM-1/PT): 0.964

Mono Adducts

Mono adducts, compounds of Formula I, wherein $R_1$ is

$CH_3CH{=}C{-}COO$
NH
C=S
$SR_2$

are prepared by reacting the paulomycin with limited amounts of mercapto containing compounds of the type used in Examples 4 to 12, $R_2SH$ or $R_3SH$, wherein $R_2$ and $R_3$ are the same as described above. By limited amounts it is meant about 1.5 equivalents. The reaction conditions are identical to those described for the production of antibiotics $273a_{2\alpha}$ and $273a_{2\beta}$, in U.S. application Serial No. 609,394, filed April 24, 1984, and are as follows.

The mercapto-compound is dissolved in phosphate buffer. The pH is adjusted to approximately 9.0; paulomycins A or B are then added under stirring (ratio of mercapto-compound to paulomycins is approximately 1.5:1). The reaction is stopped at about 30 minutes. At this time the resulting addition productions are extracted from the reaction mixture with ethly acetate or 1-butanol at the appropriate pH's (usually about 4.0). The residue obtained after removal of the solvent is a mixture of the corresponding mono and bis adducts. Purification and separation of the desired mono adduct is obtained by chromatography over silica gel using methanol-chloroform mixtures or by reverse phase chromatography in C-18 or C-8 silica gel using acetonitrile-pH 5.5 phosphate buffer mixtures. Counter double current distribution using cyclohexane-ethyl acetate-acetone-water (1:1:1:1) can be used for the purification of the mono adducts 5a to 14b. The products of the reaction can be characterized by fast atom bombardment mass spectrometry. The compounds thus produced by addition of 1 molecule of mercapto compound to paulomycins A or B have biological properties similar to those of antibiotics $273a_2$, i.e. they are active against gram-positive organisims including Staphylococcus aureus resistant to methicillin, lincosaminide and macrolide antibiotics.

Example 17 Mono adduct prepared by reacting paulomycin $A_2$ with mercapto containing compounds.

(a) Mono Adducts of Paulomycin $A_2$

N-acetyl-L-cysteine (ca. 1.5 equiv.) is dissolved in phosphate buffer. The pH is adjusted to ca. 9.0; paulomycin $A_2$ (ca. 1.0 equiv.) is then added under stirring (ratio of mercapto-compound to paulomycin. The reaction is stopped at ca 30 minutes. At this time the resulting addition products are extracted from the reaction mixture with ethyl acetate of 1-butanol at the appropriate pH's (usually ca 4.0). The residue obtained after removal of the solvent is a mixture of the corresponding mono- and bis-addult. Purification and separation of the desired mono-adduct is obtained by chromatography over silica gel using methanol-chloroform mixtures or by reverse phase chromatography in C-18 or C-8 silica gel using acetonitrile-pH 5.5 phosphate buffer mixtures. Counter double current distribution using cyclohexane-ethyl acetate-acetone-water (1:1:1:1) can be used for the purification of the mono-adducts 20a top 34a. The products of the reaction can be characterized by fast atom bombardment mass spectrometry.

Similarly other mono adducts of paulomycin $A_2$ can be prepared by subs ituting the mercapto-containing compound described in Chart III and in Examples 7 to 12 for N-acetyl-L-cysteine.

(b) Mono Adducts of Paulomycin E

N-acetyl-L-cysteine (ca 1.5 equiv.) is dissolved in phosphate buffer. The pH is adjusted to ca 9.0; paulomycin E is then added under stirring (ratio of mercapto-compound to paulomycin ca 1.5:1). The reaction is stopped at ca 30 minutes. At this time the resulting addition products are extracted from the reaction mixture with ethyl acetate of 1-butanol at the appropriate pH's (usually ca 4.0). The residue obtained after removal of the solvent is a mixture of the corresponding mono- and bis-adducts. Purification and separation of the desired mono-adduct is obtained by chromatography over silica gel using methanol-chloroform mixtures or by reverse phase chromatography in C-18 or C-8 silica gel using acetonitrile-pH 5.5 phosphate buffer mixtures. Counter double current distribution using cyclohexane-ethyl acetate-acetone-water (1:1:1:1) can be used for the purification of the mono-adducts 20a to 34a. The products of the reaction can be characterized by fast atom bombardment mass spectrometry.

Similarly other mono adducts of paulomycin E can be prepared

by substituting the other mercapto containing compounds described in chart III and Examples 7 to 12 for N-acetyl-l-cysteine.

(c) Mono adducts of Paulomycin D

N-acetyl-L-cysteine (ca 1.5 equiv.) is dissolved in phosphate buffer. The pH is adjusted to ca 9.0; paulomycin D is then added under stirring (ratio of mercapto-compound to paulomycin ca 1.5:1). The reaction is stopped at ca 30 minutes. At this time the resulting addition products are extracted from the reaction mixture with ethyl acetate of 1-butanol at the appropriate pH's (usually ca 4.0). The residue obtained after removal of the solvent is a mixture of the corresponding mono- and bis-adducts. Purification and separation of the desired mono-adduct is obtained by chromatography over silica gel using methanol-chloroform mixtures or by reverse phase chromatography in C-18 or C-8 silica gel using acetonitrile-pH 5.5 phosphate buffer mixtures. Counter double current distribution using cyclohexane-ethyl acetate-acetone-water (1:1:1:1) can be used for the purification of the mono-adducts 20a to 34a. The products of the reaction can be characterized by fast atom bombardment mass spectrometry.

Similarly other mono adducts of paulomycin D can be prepared by substituting the mercapto containing compounds described in Chart III and in Examples 7 to 12 for N-acetyl-L-cysteine.

Mixed Adducts

Mixed adducts of Formula IB are formed by reacting a bis-adduct of paulomycin, obtained by two moles of thiol in aqueous medium at pH 8.7, with a second thiol under the same conditions, with the replacement of one of the initial thio-substituents by the second, yielding a mixed bis-adduct. The group displaced is that of the dithiocarbamate (i.e., from the original isothiocyanate group). Such mixed bis-adducts, which show high antibacterial activity both *in vitro* and *in vivo*, were inaccessible previously.

The Structure of the Mixed Adducts

In the reaction between the bis(2-mercapto-ethanol) adduct of Paulomycin A ($R_T$+ ca. 16 min under the HPLC conditions on a reversed phase $C_{18}$ column described in the Experimental Section) and N-acetyl-L-cysteine, the major new peak ($R_T$+ ca. 12 min) is intermediate in value between the starting material and the bis(N-acetyl-L-cysteine)

adduct (Antibiotic $273a_{1\alpha}$) ($R_T$↓ ca. 10 min), and this intermediate value is consistent with the replacement of one $-SCH_2CH_2OH$ group in the starting material by the more polar

$-SCH_2CHCOOH$
$\quad\quad\quad|$
$\quad\quad NHCOCH_3$

group.

Similarly, in the reaction between the bis(N-acetyl-L-cysteine) adduct (Antibiotic $273a_{1\alpha}$) ($R_T$↓ ca. 10 min) and 2-mercapto-ethanol, the major new peak ($R_T$↓ ca. 12.5 min) is intermediate in value between the starting material and the bis(2-mercapto-ethanol) adduct ($R_T$↓ 16 min); and this intermediate value is consistent with the replacement of one

$-SCH_2CHCOOH$
$\quad\quad\quad|$
$\quad\quad NHCOCH_3$

group in the starting material by the less polar $-SCH_2CH_2OH$ group.

Proof that the two products described above though having very similar $R_T$↓ values, were indeed distinct entities, lay in the separation of two poorly resolved peaks by HPLC on coinjection. By negative ion FAB-MS., each compound as its mono-sodium salt, showed a molecular ion (M·) at m/z 1049 a.m.u., the value calculated for (Paulomycin A + 2-mercapto-ethanol + N-acetyl-L-cysteine, monosodium salt, $C_{41}H_{60}N_3O_{21}S_3Na$); furthermore, each showed low-mass ions at m/z 77 ($H\dot{O}CH_2CH_2S.$) and 162

$(HOOCCHCH_2S\bar{.})$
$\quad\quad\quad|$
$\quad\quad NHCOCH_3$

a.m.u., additional evidence that each contained the 2-mercapto-ethanol and N-acetyl-L-cysteine fragments, but no fragment ion revealed the environment of either substituent. The U.V. spectrum of each showed $\lambda_{max}$ 250, 274, and 328 nm characteristic in adducts of the Paulomycins of both bis- and mono- (i.e., dithiocarbamates retaining the conjugated double bond of the paulic acid ester) adducts. Similarly, the IR and N.M.R. (both $^1H$ and $^{13}C$) could be interpreted only as evidence of the absence of the $-N{=}C{=}S$ and conjugated ester double bond, and gave no indication of the relative positions of the two thio-substituents.

Under the same HPLC conditions, Antibiotic $273a_{2\alpha}$ has an $R_T$ value of 11.6 min. On reaction with 2-mercapto-ethanol, the peak corresponding

to the 273$a_{2\alpha}$ disappeared very rapidly, with the generation of a major new doublet peak of RT ca. 12 min together with a minor peak of RT ca 16 min. After 2 1/2 hours, the 16 min peak was the major product the 12 min peak having diminished markedly. The 16 min peak was coincident with that of the bis(2-mercapto-ethanol) adduct on coinjection of the two materials. The generation of this bis(2-mercapto-ethanol) product from 273$a_{2\alpha}$ demonstrates that the thio-component of the dithiocarbamate group of an adduct is displaced readily by a second thiolate anion, and thereby demonstrates that the reaction involved with a bis-adduct occurs as illustrated in Chart IV.

The doublet peak of ca $R_T$ 12 min, seen at the earlier time of assay and at which point the starting 273$a_{2\alpha}$ has disappeared, is a mixture of components of RT 12.12 and 12.35 min. The former is not distinguished from the mixed bis-adduct derived from the reaction between the bis(2-mercapto-ethanol) adduct and N-acetyl-L-cysteine, and must be derived from the addition of 2-mercapto-ethanol to the conjugated double bond of the ester of 273$a_{2\alpha}$. The second intermediate, of RT 12.35 min,.must be that of displacement of the N-acetyl-L-cysteine anion by the 2-mercapto-ethanol with retention of the conjugated double bond of 273$a_{2\alpha}$ (11.6 min). The course of the reaction between 273$a_{2\alpha}$ and 2-mercapto-ethanol is as illustrated in Chart VII.

<u>Example 18</u> Reaction of Paulomycins with 2-mercapto-ethanol

2-Mercapto-ethanol (992 mg, 0.89 ml, 10 equiv) is dissolved in aqueous phosphate buffer (0.05 M, pH 7.85, 100 ml), and the solution is adjusted to pH 8.7 by the addition of aqueous sodium hydroxide (N). Paulomycin A (1.0 g, 1 equiv. solid) is added to this vigorously stirred solution and dissolves completely within 10 min. Analytical HPLC shows the presence of only one product, and the complete disappearance of Paulomycin A.

The stirred reaction solution is acidified with aqueous hydrochloric acid (N) of pH 5.5, causing the precipitation of the product, which is collected by filtration and is washed with water. HPLC analysis shows the product to be free of excess 2-mercapto-ethanol. A solution of the solid in ethyl acetate is dried sodium sulfate; removal of the solvent in vacuo. The residue is dissolved in acetone and this solution, under stirring, is diluted with ether and Skellysolve B,

giving a colorless, amorphous precipitate which is dried in vacuo at room temperature.

This bis-adduct is characterized by fast atom bombardment mass spectrometry (see Table 2).

Utilizing a procedure similar to that described in Example 18, but substituting Paulomycins $A_2$, B, D and E for Paulomycin A there are obtained the corresponding bis-adducts.

Example 19 Reaction of the bis(2-mercapto-ethanol) adduct of Paulomycins with N-acetyl-L-cysteine - the mixed (2-mercapto-ethanol)-N-acetyl-L-cysteine adduct

The bis-(2-mercapto-ethanol) adduct of Paulomycin A (900 mg, 1 equiv, solid) is added to a vigorously stirred solution of N-acetyl-L-cysteine (1.56 g, 10 equiv.) in aqueous pH 7.85 phosphate buffer (0.05 M, 100 ml), which has been adjusted to pH 8.7 by the addition of aqueous sodium hydroxide (N); all of the solid has dissolved within 15 min. Analytical HPLC examination of an aliquot of the reaction solution after 1 1/2 hours showed starting material (minor, RT ca 16 min) and product (major, RT ca 12 min); after 24 hours, very little starting material remains, and a minor zone, $R_T$+ ca 10 min, is present.

On acidification of the reaction solution with aqueous hydrochloric acid (2N) to pH 3.0, a colorless flocculent precipitate is formed. Extraction with ethyl acetate, drying with sodium sulfate, and removal of the solvent in vacuo gives an amorphous solid, which is dissolved in acetonitrile (5 ml), injected onto a partisil 40 ODS-3 $C_{18}$ reversed phase column, and eluted with a mixture of aqueous phosphate buffer (pH 5.5) and acetonitrile using a gradient of 15% acetonitrile to 40%. Elution is monitored at 220 nm. Fractions corresponding to the peaks are collected, resulting in pools which are assayed by analytical HPLC. These fractions re acidified separately to pH 3 with aqeuous hydrochloric acid (N), extracted with ethyl acetate, dried sodium sulfate, and the solvent removed in vacuo, giving colorless amorphous residues.

Fraction A, RT ca 10 min, separates from acetone solution on the addition of ether as a colorless, amorphous solid, identified by HPLC, U.V. spectrum, and FAB-MS as antibiotic $273a_1$.

Fraction B, RT+ ca 12 min, is dissolved in ethyl acetate, and a solution of sodium 2-ethylhexanoate in the same solvent is added with stirring, producing a precipitate of the mono-sodium salt of the mixed 2-mercapto-ethanol-N-acetyl-L-cysteine adduct, characterized by FAB-MS (see Table 2).

Utilizing a procedure similar to that described in Example 19 but substituting the corresponding bis-adducts of Paulomycins A, $A_2$, B, D and E for the bis-adduct of Paulomycin A there is obtained the corresponding mixed adducts.

<u>Example 20</u> Reaction of Antibiotic $273a_{1\alpha}$ with 2-mercapto-ethanol- the mixed N-acetyl-L-cysteine - (2-mercapto-ethanol) adduct

In a manner analogous entirely to Example 19, the di-sodium salt of Antibiotic $273a_{1\alpha}$ (1.0 g, 1 equiv., solid) is added to a solution of 2-mercapto-ethanol (675 mg, 0.61 ml, 10 equiv) in aqueous phosphate buffer (0.05 M, pH 7.85, 100 ml) which has been adjusted to pH 8.7 by the addition of aqueous sodium hydroxide (N). The reaction is followed by analytical HPLC. By two hours, very little $273a_{1\alpha}$ remains, and the major product shows RT ca 13 min. Following acidification to pH 3, extraction with ethyl acetate, and removal of the solvent in vacuo, the residue is dissolved in a minimal volume of acetonitrile and chromatographed as for Example 15. The major eluate is isolated as before, and converted into the mono-sodium salt, characterized by FAB-MS (see Table 2).

<u>Example 21</u> Reaction of the bis(2-mercapto-ethanol) adduct of Paulomycin A with thiomalic acid - the mixed (2-mercapto-ethanol)-thiomalic acid adduct

In a manner analogous to that used in Examples 19 and 20, the bis-(2-mercapto-ethanol) adduct of Paulomycin A (6.0 g, 1 equiv. solid) is added to a vigorously stirred solution of thiomalic acid (19.1 g, 20 equiv) in aqueous phosphate buffer (0.05 M, pH 7.85, 150 ml) which has been adjusted by the addition of aqueous sodium hydroxide (N) to pH 8.7. After 24 hours, analytical HPLC shows a major product of RT+ ca 12 min.

The pH of the reaction solution is adjusted to 5.5 by the addition of concentrated hydrochloric acid, and the resulting solution is pumped

onto two cartridges in sequence (C-18, using the Waters "Prep 500: chromatogram) which has been equilibrated with 156 of acetonitrile in aqueous phosphate buffer (0.05 M, pH 5.5), monitoring the eluate at 254 nm. After eluting the column for 15 mins, a gradient elution is introduced whereby the percentage of acetonitrile is increased to 40 after an additional 30 min, and then held at this composition critically.

The appropriate fractions are determined by analytical HPLC, pooled, acidified to pH 3 with aqueous hydrochloric acid (N), extracted with ethyl acetate, and the extract dried ($Na_2SO_4$). This solution is concentrated in vacuo to a small volume and converted into the di-sodium salt by reaction with a solution of sodium 2-ethylhexanoate, which is removed by filtration, washed with ethyl acetate-Skellysolve B, and dried at room temperature in vacuo. This product is characterized by FAB-MS (see Table 2).

Utilizing a procedure similar to that discribed in Example 21 but substituting the bis-(2-mercapto-ethanol) adducts of Paulomycins $A_2$, B, D and E for the bis-(2-mercapto-ethanol) adduct of Paulomycin A there is obtained the corresponding mixed (2-mercapto-ethanol)-thiomalic acid adducts.

Example 22A Reaction of the bis-thiomalic acid adduct of Paulomycin A with 2-mercapto-ethanol- the mixed thiomalic acid -(2-mercapto-ethanol adduct)

The bis-thiomalic acid adduct of Paulomycin A (5.0 g, solid, 1 equiv) is added to a solution of 2-mercapto- thanol) 3.6 g, 10 equiv) in aqueous phosphate buffer (100 ml, pH 7.85) which has been adjusted to pH 8.7 by the addition of aqueous sodium hydroxide (N). The course of the reaction is followed by analytical HPLC; the product is isolated following the scheme of Example 22, and converted into the disodium salt.

The compounds prepared in Examples 18 through 22 were characterized by fast atom bombardment mass spectroscopy (See Table 2).

TABLE 2

| Starting Material | Reagent | Product | Molecular Formula | Molecular Weight (by FAB - MS) |
|---|---|---|---|---|
| Paulomycin A | $HSCH_2CH_2OH$ | 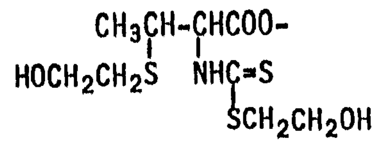 | $C_{36}H_{48}N_2O_{18}S$ | 860 |
| Bis(2-mercapto-ethanol)adduct of Paulomycin A | N-acetyl-L-cysteine | 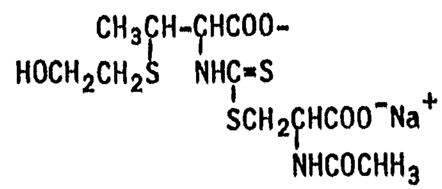 | $C_{41}H_{60}N_3O_{21}S_3Na$ | 1049 |
| Antibiotic $273a_1\alpha$ | $HSCH_2CH_2OH$ | 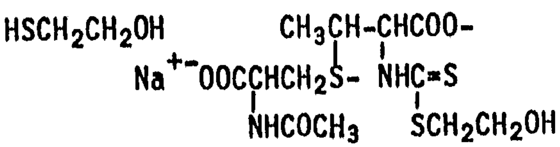 | $C_{41}H_{60}N_3O_{21}S_3Na$ | 1049 |
| Bis(2-mercapto-ethanol)adduct of Paulomycin A | Thiomalic acid | 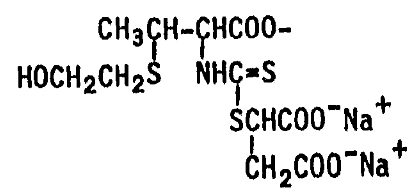 | $C_{40}H_{56}N_2O_{22}S_3Na_2$ | 1058 |
| Bis-thiomalic acid adduct of Paulomycin A | $HSCH_2CH_2OH$ | 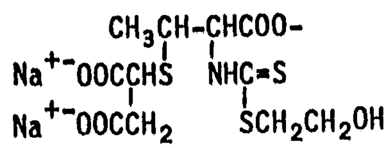 | $C_{40}H_{56}N_2O_{22}S_3Na_2$ | 1058 |

$CH_3CH-CHCOO-$
$R_2S$ $NHC=S$
$SR_3$

| | | | | | | |
|---|---|---|---|---|---|---|
| | | $R_2=CH_2CH_2OH$<br>$R_3=CH_2CH_2OH$ | $R_2=CH_2CHCOOH$<br>$NHCOCH_3$<br>$R_3=CH_2CH_2OH$ | $R_2=CH_2CH_2OH$<br>$R_3=CH_2CHCOOH$<br>$NHCOCH_3$ | $R_2=CH_2CH_2OH$<br>$R_3=CHCOOH$<br>$CH_2COOH$ | $R_2=CHCOOH$<br>$CH_2COOH$<br>$R_3CH_2CH_2OH$ |
| <u>Staphylococcus</u> | | | | | | |
| <u>aureus</u> U.C. | 76 | 0.31 | -- | -- | -- | -- |
| | 6675 | -- | 0.5 | 0.25 | 0.125 | 1.0 |
| | 9218 | -- | 0.25 | 0.25 | -- | 0.5 |
| | 3665 | -- | 0.5 | 0.5 | 0.125 | 0.5 |
| | 6685 | 0.31 | 0.5 | 0.5 | <0.06 | 0.5 |
| | 6690 | 0.31 | -- | -- | -- | -- |
| <u>Streptococcus</u> | | | | | | |
| <u>pneumoniae</u> | | | | | | |
| U.C. | 41 | 1.25 | 0.25 | 0.25 | <0.06 | 0.125 |
| <u>Streptococcus</u> | | | | | | |
| <u>faecalis</u> | | | | | | |
| U.C. | 694 | -- | 0.5 | 0.5 | -- | 2.0 |

4037

# Antibacterial Activity, in Vivo, of Mixed Adducts of Paulomycin A

$R_1$ = 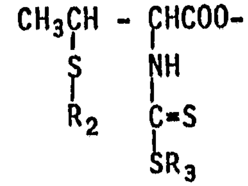

($CD_{50}$ values in mg/kilo, mouse, dosed subcutaneously, infected experimentally with *Staphylococcus aureus* U.C. 6685)

| | | | | |
|---|---|---|---|---|
| $R_2=CH_2CH_2OH$ | 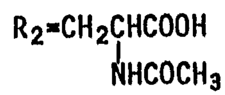 | $R_2=CH_2CH_2OH$ | 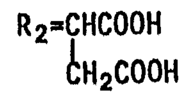 | $R_2=CH_2CH_2OH$ |
| $R'_3=CH_2CH_2OH$ | $R_3=CH_2CH_2OH$ | 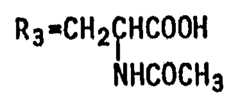 | $R_3=CH_2CH_2OH$ | 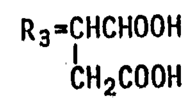 |
| 14.5 | 8.0 | 15.3 | 44 | -- |

THE IN VITRO ACTIVITY OF PAULOMYCIN A AND PAULOMYCIN $A_2$ AGAINST AEROBIC BACTERIA

| | | MINIMAL INHIBITORY CONCENTRATION (μg/ml) | |
|---|---|---|---|
| Organism Name | UC# | Paulomycin $A_2$ | Paulomycin A |
| | | pH 6 | pH 6 |
| Enterobacter cloacae | 9381 | >64 | >64 |
| Enterobacter cloacae | 9382 | >64 | >64 |
| Klebsiella oxytoca | 9383 | >64 | >64 |
| Klebsiella oxytoca | 9384 | >64 | >64 |
| Escherichia coli | 9379 | >64 | >64 |
| Escherichia coli | 9380 | >64 | >64 |
| Escherichia coli | 311 | >64 | >64 |
| Staphylococcus aureus | 6675 | 0.12 | 0.12 |
| Staphylococcus aureus | 3665 | 0.12 | 0.12 |
| Staphylococcus aureus | 6685 | 0.12 | 0.12 |
| Streptococcus faecalis | 694 | 0.25 | 0.25 |
| Klebsiella pneumoniae | 58 | >64 | >64 |
| Pseudomonas aeruginosa | 9191 | >64 | >64 |
| Pseudomonas aeruginosa | 6432 | >64 | >64 |
| Serratia marcescens | 6888 | >64 | >64 |
| Citrobacter freundii | 3507 | >64 | >64 |
| Proteus vulgaris | 30264 | 32 | 16 |

NOTE: "UC" is a registered trademark of The Upjohn Company.

The conditions of the above test are as follows:

The antibacterial assay is a standard microplate agar assay using PYG agar, pH 6. PYG agar consists of the following ingredients:

| | |
|---|---|
| Peptone | 10 g./1. |
| Yeast extract | 5 g./1. |
| Glucose | 1 g./1. |
| Agar | 15 g./1. |
| Distilled water, q.s. | 1 1. |

---

The MIC is determined by standard methods. The inocula are overnight cultures of the test organisms, diluted so that the final population contains approximately $10^5$ cells/ml. The agar plates are incubated at 28°C to 37°C for 24 hrs. The lowest antibiotic concentration which allows no growth=MIC or minimum inhibitory concentration.

THE IN VITRO ACTIVITY OF PAULOMYCIN A AND PAULOMYCIN D AGAINST AEROBIC BACTERIA

| | | MINIMAL INHIBITORY CONCENTRATION (µg/ml) | |
|---|---|---|---|
| Organism Name | UC# | Paulomycin D | Paulomycin A |
| | | pH 6 | pH 6 |
| *Enterobacter cloacae* | 9381 | >64 | >64 |
| *Enterobacter cloacae* | 9382 | >64 | >64 |
| *Klebsiella oxytoca* | 9383 | >64 | >64 |
| *Klebsiella oxytoca* | 9384 | >64 | >64 |
| *Escherichia coli* | 9379 | >64 | >64 |
| *Escherichia coli* | 9380 | >64 | >64 |
| *Escherichia coli* | 311 | >64 | >64 |
| *Staphylococcus aureus* | 6675 | 1 | 0.12 |
| *Staphylococcus aureus* | 3665 | 0.5 | 0.12 |
| *Staphylococcus aureus* | 6685 | 0.5 | 0.12 |
| *Streptococcus faecalis* | 694 | 1 | 0.25 |
| *Klebsiella pneumoniae* | 58 | >64 | >64 |
| *Pseudomonas aeruginosa* | 9191 | >64 | >64 |
| *Pseudomonas aeruginosa* | 6432 | >64 | >64 |
| *Serratia marcescens* | 6888 | >64 | >64 |
| *Citrobacter freundii* | 3507 | >64 | >64 |
| *Proteus vulgaris* | 30264 | >64 | 16 |

NOTE: "UC" is a registered trademark of The Upjohn Company.

The conditions of the above test are as follows:

The antibacterial assay is a standard microplate agar assay using PYG agar, pH 6. PYG agar consists of the following ingredients:

| | |
|---|---|
| Peptone | 10 g./1. |
| Yeast extract | 5 g./1. |
| Glucose | 1 g./1. |
| Agar | 15 g./1. |
| Distilled water, q.s. | 1 1. |

---

The MIC is determined by standard methods. The inocula are overnight cultures of the test organisms, diluted so that the final population contains approximately $10^5$ cells/ml. The agar plates are incubated at 28°C to 37°C for 24 hrs. The lowest antibiotic concentration which allows no growth=MIC or minimum inhibitory concentration.

THE IN VITRO ACTIVITY OF PAULOMYCIN A AND PAULOMYCIN E AGAINST AEROBIC BACTERIA

| | | MINIMAL INHIBITORY CONCENTRATION (μg/ml) | |
|---|---|---|---|
| Organism Name | UC# | Paulomycin E | Paulomycin A |
| | | pH 6 | pH 6 |
| Enterobacter cloacae | 9381 | >64 | >64 |
| Enterobacter cloacae | 9382 | >64 | >64 |
| Klebsiella oxytoca | 9383 | >64 | >64 |
| Klebsiella oxytoca | 9384 | >64 | >64 |
| Escherichia coli | 9379 | >64 | >64 |
| Escherichia coli | 9380 | >64 | >64 |
| Escherichia coli | 311 | >64 | >64 |
| Staphylococcus aureus | 6675 | 1 | 0.12 |
| Staphylococcus aureus | 3665 | 1 | 0.12 |
| Staphylococcus aureus | 6685 | 1 | 0.12 |
| Streptococcus faecalis | 694 | 1 | 0.25 |
| Klebsiella pneumoniae | 58 | >64 | >64 |
| Pseudomonas aeruginosa | 9191 | >64 | >64 |
| Pseudomonas aeruginosa | 6432 | >64 | >64 |
| Serratia marcescens | 6888 | >64 | 64 |
| Citrobacter freundii | 3507 | >64 | >64 |
| Proteus vulgaris | 30264 | >64 | 16 |

NOTE: "UC" is a registered trademark of The Upjohn Company.

The conditions of the above test are as follows:

The antibacterial assay is a standard microplate agar assay using PYG agar, pH 6. PYG agar consists of the following ingredients:

| | |
|---|---|
| Peptone | 10 g./l. |
| Yeast extract | 5 g./l. |
| Glucose | 1 g./l. |
| Agar | 15 g./l. |
| Distilled water, q.s. | 1 l. |

---

The MIC is determined by standard methods. The inocula are overnight cultures of the test organisms, diluted so that the final population contains approximately $10^5$ cells/ml. The agar plates are incubated at 28°C to 37°C for 24 hrs. The lowest antibiotic concentration which allows no growth=MIC or minimum inhibitory concentration.

THE IN VITRO ACTIVITY OF PAULOMYCIN AND ANTIBIOTIC $273a_1$ AGAINST ANAEROBIC BACTERIA

| | | MINIMAL INHIBITORY CONCENTRATION (μg/ml) | | |
|---|---|---|---|---|
| | | $273a_1$ | | Paulomycin |
| Organism | UC# | pH 6 | pH 7 | pH 6 |
| *Bacteroides fragilis* | 6613 | 5 | 10 | 2.5 |
| *Bacteroides fragilis* | 6428 | 5 | 10 | 2.5 |
| *Bacteroides distasonis* | 6518 | 0.31 | 2.5 | ≤0.16 |
| *Bacteroides thetaiotaomicron* | 6512 | 20 | 40 | 10.0 |
| *Bacteroides melaninogenicus* | 6523 | 1.25 | 5 | 0.31 |
| *Fusobacterium nucleatum* | 6324 | 2.5 | 2.5 | 0.62 |

| | | MINIMAL INHIBITORY CONCENTRATION (μg/ml) | | |
|---|---|---|---|---|
| | | $273a_1$ | | Paulomycin |
| *Fusobacterium necrophorum* | 6568 | 5 | 40 | 5.0 |
| *Eubacterium lentum* | 6327 | ≤0.16 | 1.25 | ≤0.16 |
| *Veillonella alcalescens* | 6323 | 80 | 160 | 40.0 |
| *Peptostreptococcus variabilis* | 6320 | ≤0.16 | 0.62 | 0.16 |
| *Clostridium difficile* | 6857 | 0.31 | 1.25 | 0.31 |
| *Clostridium perfringens* | 6509 | 0.31 | 5 | 0.31 |
| *Clostridium sporogenes* | 6308 | ≤0.16 | 2.5 | 0.31 |
| *Proprionibacterium acnes* | 6564 | 0.31 | 2.5 | ≤0.16 |

The procedure for the above test is as follows:

Serial two-fold dilutions of drug are prepared in 1.0 ml volumes of Schaedler Broth, and 9.0 ml of molten (47°C) Wilkens-Chalgren Agar Medium, infra, is added to the antibiotic-supplemented broth. After mixing with the antibiotic, the agar is poured into 100 mm x 20 mm petri dishes. The dishes are allowed to stand on the bench overnight prior to inoculation.

Cultures are streaked on Wilkens-Chalgren Agar, and grown for 48 hours at 37°C in a BBL Anaerobe Jar. Growth from the plate is harvested, and a cell suspension is made in Schaedler broth to equal the turbidity of a 0.5 McFarland Standard ($10^8$ cells/ml). The suspension is pipetted into wells of a Steers replicator, and approximately 1-2 μl is delivered to the surface of the agar plates. After allowing a few minutes for the inoculum to dry, the plates are

placed in a BBL Anaerobe Jar (atmosphere of 85% N, 10% H, 5% $CO_2$) and incubated at 37°C for 72 hours.

The Minimal Inhibitory Concentration (MIC) is read as the least amount of drug that inhibits growth. A very faint film of growth, or <3 colonies is considered negative.

Preparation of Wilkins-Chalgren Agar Medium

Dispense the following ingredients and dissolve in 1000 ml distilled water. The pH should be 7.0 - 7.2.

| | |
|---|---|
| Trypticase | 10 g |
| Gelysate | 10 g |
| Yeast Extract | 5 g |
| Glucose | 1 g |
| NaCl | 5 g |
| L-Arginine-Free Base | 1 g |
| Pyruvic Acid-Sodium Salt | 1 g |
| Agar | 15 g |

Add Heme and Vitamin $K_1$ solutions to yield final concentrations of 5 μg/ml Hemin and 0.5 g/ml Vitamin $K_1$.

Autoclave at 121°C for 15 minutes aerobically.

Heme Stock - 0.5 g Hemin + 10 ml 1 N NaOH + 990 ml $H_2$.

Autoclave at 121°C for 12 minutes.

Add 10 ml Heme Stock per liter of medium.

Vitamin K Stock - .05 ml Vitamin $K_1$ solution + 20 ml 95% ethanol.

Filter sterilize.

Add 0.2 ml Vitamin K Stock per liter of medium.

Thus, the antibacterially-active compounds of the present invention can be used for the same antibacterial purposes as paulomycin A and B. For example, the compounds of the invention can be used alone or in combination with other antibiotic agents to prevent the growth of, or reduce the number of the above described susceptible bacteria in many environments. For example, they can be used as disinfectants on various dental and medical equipment contaminated with Staphylococcus aureus. Further, they are useful in wash solutions for sanitation purposes, as in the washing of hands and in the cleaning of equipment, floors, or furnishings or contaminated rooms or laboratories; they are also useful as an industrial preservative, for example, as a bacteriostatic rinse for laundered

clothes and for impregnating papers and fabrics; and they a e useful for suppressing the growth of sensitive organisms in plate assays and other microbiological media. They also can be used as feed supplements to promote the growth of animals, for example, mammals, birds, fish, and reptiles.

The compounds of the subject invention are useful as antibacterial agents in suitable composi:ions. These compositions are preferably presented for administration to humans and animals in unit dosage forms, such as tablets, capsules, pills, powders, granules, sterile parenteral solutions or suspensions, and oral solutions or suspensions, and oil-water emulsions containing suitable quantities of the active compound in the form of the free base, or its pharmacologically acceptable salts.

The tribasic and tri acid salts are particularly desirable compounds because of their long term stability.

For oral administration, either solid or fluid unit dosage forms can be prepared. For prep ring solid compositions such as tablets, the principal active ingredient is mixed with conventional ingredients such as talc, magnesium, stearate, dicalcium phosphate, magnesium aluminum silicate, calcium sulfate, starch, lactose, acacia, methylcellulose, and functionally similar materials as pharmaceutical diluents or carriers. The tablets an be laminated or otherwise compounded to provide a dosage form affording the advantage of prolonged or delayed action or predetermined successive action of the enclosed medication. For example, the tablet can comprise an inner dosage and an outer dosage component, the latter being in the form of an envelope over the former. The two components can be separated by an en eric layer which serves to resist distintegration in the stomach and permits the inner component to pass intact into the duodenum or to be delayed in release. A variety of materials can be used for such enteric layers or coatings, such materials including a number of polymeric acids or mixture of polymeric acids with such materials as shellac, cetyl alcohol, cellulose acetate phthalate, styrene maleic acid copolymer and the like. Alternatively, the two component system can be utilized for preparing tablets containing two or more incompatible active ingredients. Wafers are prepared in the same manner as tablets, differing

only in shape and the inclusion of sucrose or other sw etener and flavor. In their simplest embodiment, capsules, like tablets, are prepared by mixing the compound of the formulation with an inert pharmaceutical diluent and filling the mixture into a hard capsule of appropriate size. In another embodiment, capsules are prepared by filling hard gelatin capsules with polymeric acid coated beads containing the active compound. Soft gelatin capsules are prepared by machine encapsulation of a slurry of the active compound with an acceptable vegetable oil, light liquid petrolatum or other inert oil.

Fluid unit dosage forms for oral administration such as syrups, elixirs, and suspensions can be prepared. The water-soluble forms of the active compound can be dissolved in an aqueous vehicle together with sugar, aromatic flavoring agents and preservatives to form a syrup. An elixir is prepared by using a hydro-alcoholic (ethanol) vehicle with suitable sweeteners such as sucrose together with an aromatic flavoring agent. Suspensions can be prepared of the insoluble forms with a syrup vehicle with the aid of a suspending agent such as acacia, tragacanth, methylcellulose and the like.

Topical ointments can be prepared by dispersing the active compound in a suitable ointment base such as petrolatum, lanolin, polyethylene glycols, mixtures thereof, and the like. Advantageously, the compound is finely divided by means of a colloid mill utilizing light liquid petrolatum as a levitating agent prior to dispersing in the ointment base. Topical creams and lotions are prepared by dispersing the compound in the oil phase prior to the emulsification of the oil phase in water.

For parenteral administration, fluid unit dosage forms are prepared utilizing the active compound and a sterile vehicle, water being preferred. The active compound, depending on the form and concentration used, can be either suspended or dissolved in the vehicle. In preparing solutions, a water-soluble form of the active compound can be dissolved in water for injection and filter sterilized before filling into a suitable vial or ampul and sealing. Advantageously adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying

vial of water for injection is supplied to reconstitute the powder prior to use. Parenteral suspensions are prepared in substantially the same manner except that the active compound is suspended in the vehicle instead of being dissolved and sterilization cannot be accomplished by filtration. The active compound can be sterilized by exposure to ethylene oxide before suspending the sterile vehicle. Advantageously, a surfactant or wetting agent is included in the composition to facilitate uniform distribution of the active compound.

The term unit dosage form as used in the specification and claims refers to physically discrete units suitable as unitary dosages for human subjects and animals, each unit containing a predetermined quantity of active material calculated to produce the desired therapeutic effect in association with the required pharmaceutical diluent, carrier or vehicle. The specifications for the novel unit dosage forms of this invention are dictated by and directly dependent on (a) the unique characteristics of the active material and the particular therapeutic effect to be achieved, and (b) the limitations inherent in the art of compounding such an active material for therapeutic use in humans and animals, as disclosed in detail in this specification, these being features of the repesent invention. Examples of suitable unit dosage forms in accord with this invention are tablets, capsules, pills, troches, suppositories, powder packets, granules, wafers, cachets, teaspoonfuls, tablespoonfuls, dropperfuls, ampuls, vials, segregated multiples of any of the foregoing, and other forms as herein described.

In addition to the administration of the active compound as the principal active ingredient of compositions for the treatment of the conditions described herein., the said compound can be included with other types of compounds to obtain advantageous combinations of properties. Such combinations include the active compound with antibiotics such as spectinomycins, chloramphenicol, novobiocin, dihydronovobiocin, tetracyclines (e.g., tetracycline, oxytetracycline and chlortetracycline), penicillins, erythromycin, kanamycin, streptomycin, neomycin, polymyxin, bacitracin, nystatin, filipin, fumagillin and endomycin to broaden the bacterial spectrum of the composition and for synergistic action against particular bacteria; steroids having anti-inflammatory activity such as hydrocortisone, prednisolone, 6α-methylprednisolone, 6α-fluoro-

prednisolone and the like; analgesics such as aspirin, sodium salicylate (acetylsalicyclic acid)-anhydride, N-acetyl-p-aminophenyl and salicylamide; antihistamines, such as chlorpheniramine maleate, diphenylhydramine, promethazine, pyrathiazine, and the like; sulfas, such as sulfadiazine, sulfamethazine, sulfamerazine sulfacetamide, sulfadimethyloxazole, sulfamethizole, and the like; antifungals, such as undecylenic acid, sodium propionate, salicylanilide, sodium caprylate, and hexetidine; and the vitamins.

The dosage of the active compound for treatment depends on route of administration; the age, weight, and condition of the patient; and the particular disease to be treated. A dosage schedule of from about 15 to 500 mg , 1 to 4 times daily (every six hours), embraces the effective range for the tratment of most conditions for which the compositions are effective. For children, the dosage is calculated on the basis of 15 to 30 mg/kg/day to be administered every six hours. The selection of suitable patients and dosages for the treatment of humans and animals with the compounds of this invention is readily undertaken by an ordinarily skilled physician or veterinarian.

The active compound is compounded with a suitable pharmaceutical carrier in unit dosage form for convenient and effective administration. In the preferred embodiments of this invention, the dosage units contain the compound in: 15, 30, 50, 125, 250, and 500 mg amounts for systemic treatment; in 0.25, 0.5, 1, 2 and 5% amounts for topical or localized treatment; and 5 to 65% w/v for parenteral treatment. The dosage of compositions containing the active compound and one or more other active ingredients is to be determined with reference to the usual dosage of each such ingredient.

FORMULAE

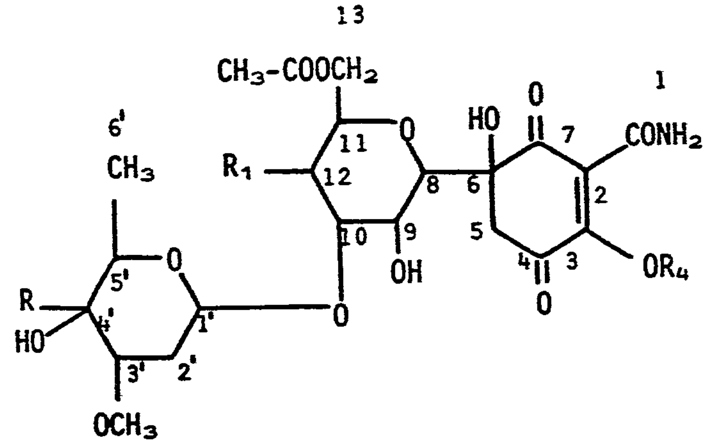

I

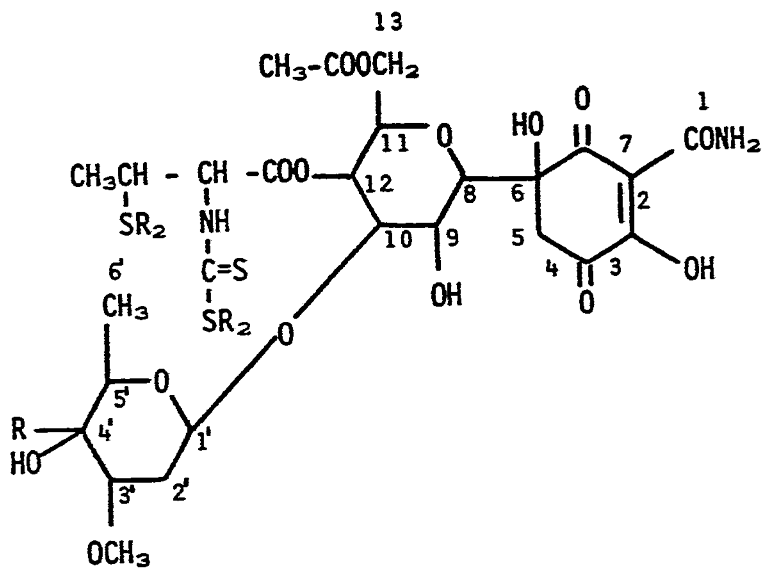

Ib

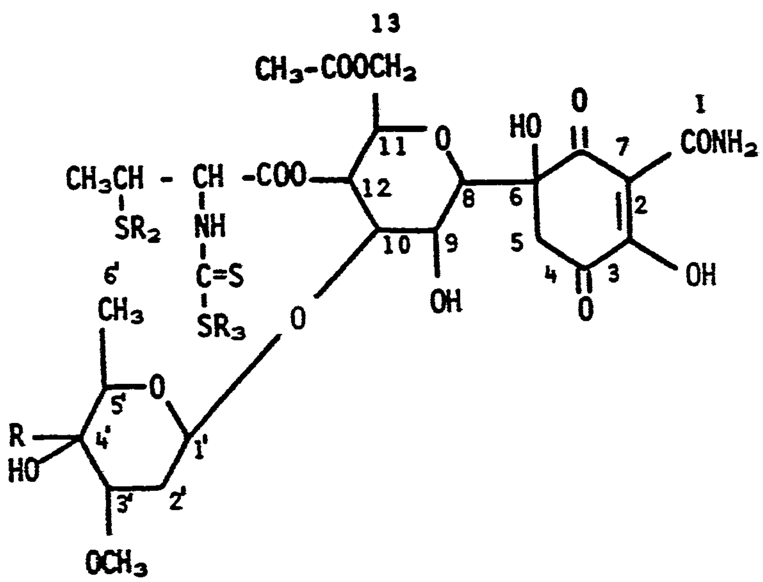

Ie

CHART I

The structure of paulomycins A, B, $A_2$, D and E are represented by 1, 2, 3, 4 and 5, respectively.

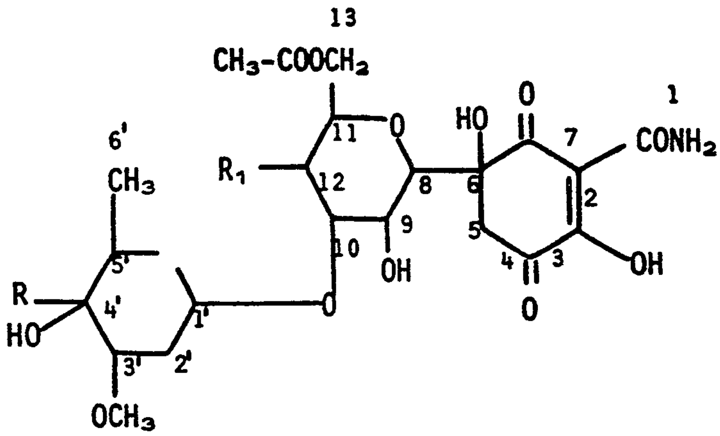

I'

R

1. Paulomycin A: 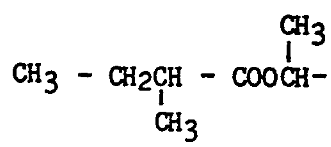

2. Paulomycin B: 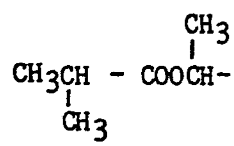

3. Paulomycin $A_2$: 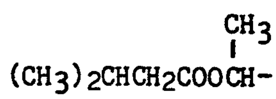

4. Paulomycin D: 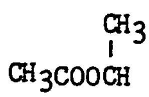

5. Paulomycin E: 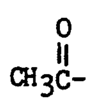

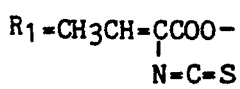

CHART I (continued)

The structure of antibiotic $273a_1\alpha$ and $273_1\beta$ are represented by 3 and 4, respectively.

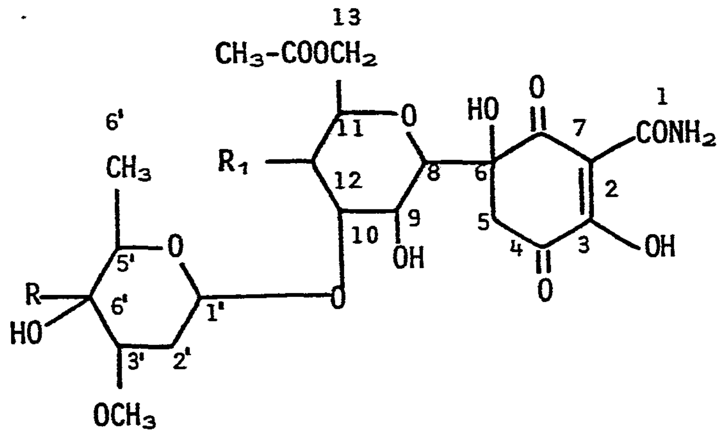

Ic

$273_1\alpha$: 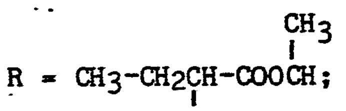 ; $273a_1\beta$: 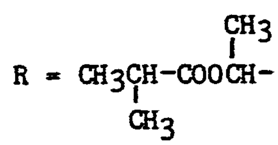

$R_1$ = 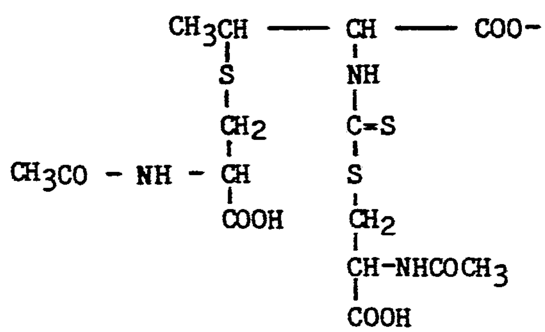

CHART II

$R_2SH$ or $R_3SH$

5 $R_2$ =-$CH_2COOH$ (mercaptoacetic acid)

6. $R_2$ =-$CH_3CHCOOH$ (mercaptopropionic acid)

7. $R_2$ = COOH (thiomalic acid)
CH-
$CH_2$
COOH

8. $R_2$ = -$CH_2CHCOOH$ (cysteine)
$NH_2$

9. $R_2$ = $CH_2CH_2CHCOOH$ (homocysteine)
$NH_2$

10. $R_2$ = $H_2N$-CH-$CH_2CH_2CONHCHCONHCH_2COOH$ (glutathione)
COOH $CH_2$-

11. $R_2$ =

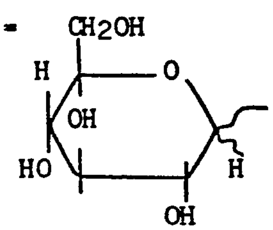

12. $R_2$ = $CH_2$-
CHOH
$CH_2OH$

13. $R_2$ = $CH_2$-
CHOH
CHOH
CHOH
$CH_2OH$

14. $R_2$ = $CH_2$-
CHOH
CHOH
CHOH
CHOH
$CH_2OH$

15. $R_2$=-$CH_2CH_2OH$

CHART III

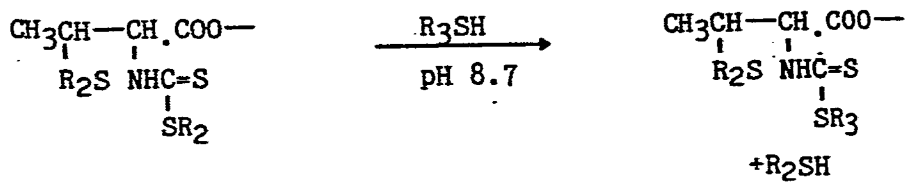


CHART IV

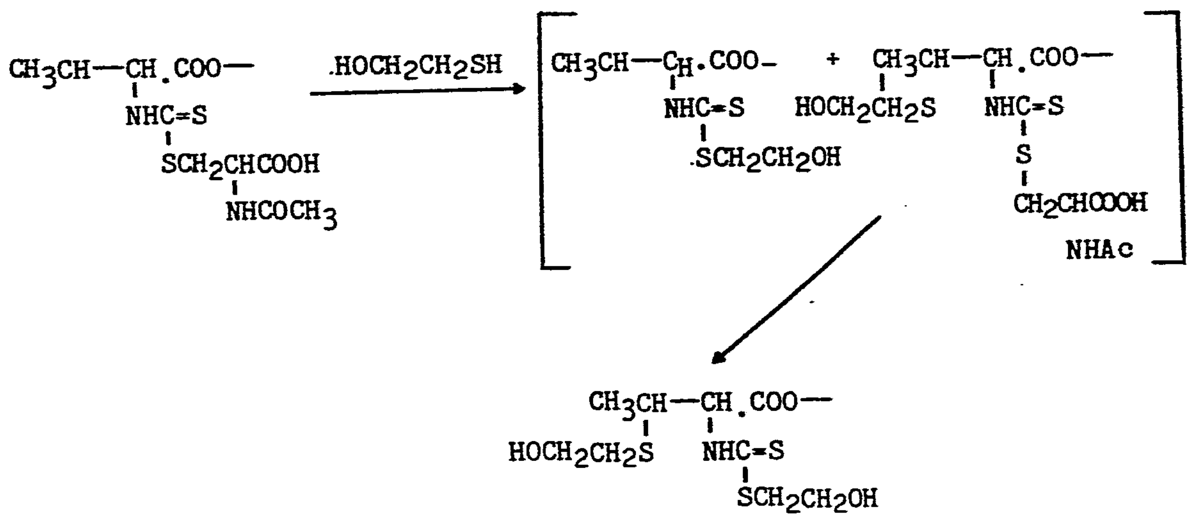

## CLAIMS

1. An antibacterially active compound of the formula I:

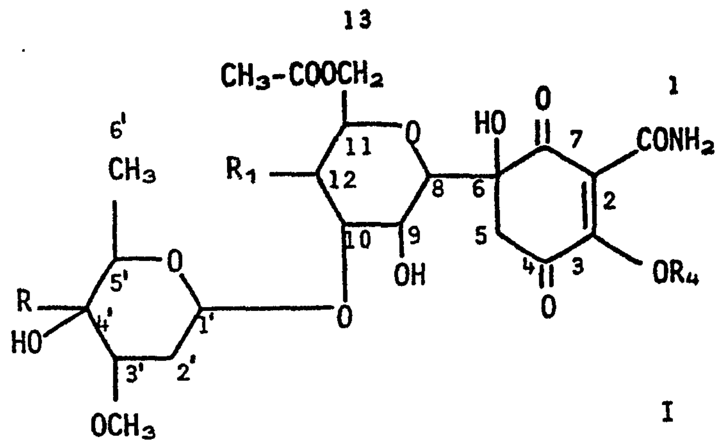

I

or a pharmacologically acceptable salt thereof;

wherein R is

(a) 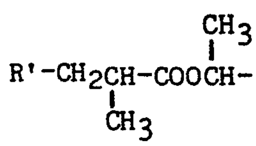

(b) 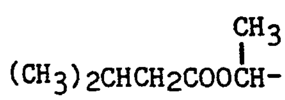

(c) 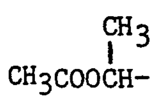

(d) 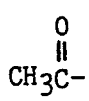

wherein R' is hydrogen or methyl;

wherein $R_1$ is

(a) 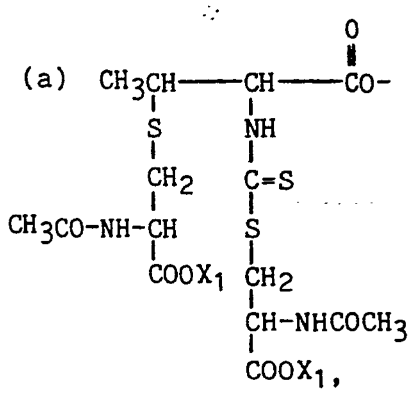

(b) 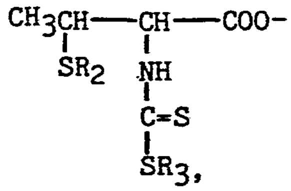

(c) 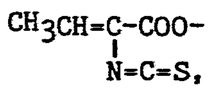 or

(d) 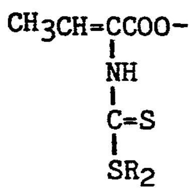

wherein $X_1$ is

(a) hydrogen,

(b) $C_1$ to $C_{12}$ alkyl, branched or straight chain,

(c) a pharmacologically acceptable cation;

wherein $R_2$ and $R_3$ are the same or different and are selected from the group

(a) $-CH_2COOX_2$,

(b) 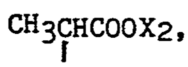

(c) 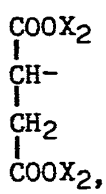

(d) 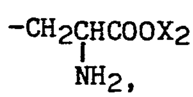

(e) 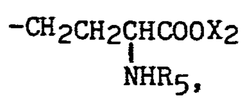

(f) 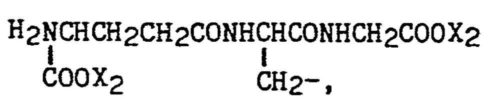

(g) 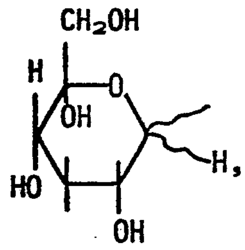

(h) 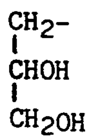 , or

(i) 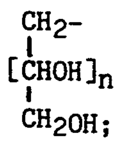

(j) $-CH_2CH_2OH$

wherein n is 3 or 4; and

wherein $R_4$ is hydrogen or a pharmacologically acceptable cation;

wherein $R_5$ is hydrogen or acetyl;

wherein $X_2$ is hydrogen or a pharmacologically acceptable cation, with the proviso that when R is

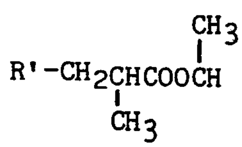

$R_1$ cannot be

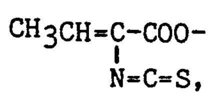 , or

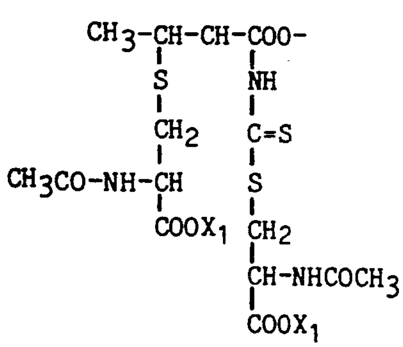

when $X_1$ is hydrogen;

or

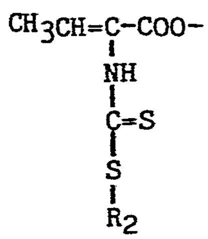

2. A compound of claim 1 having the formula:

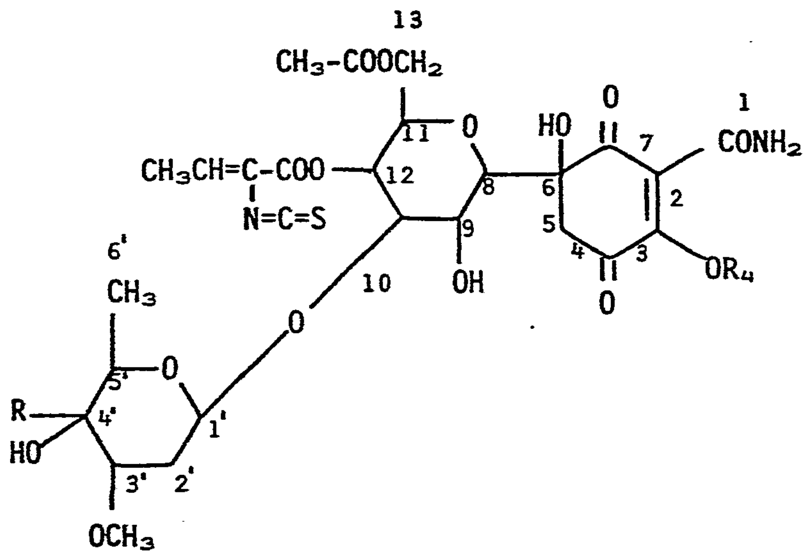

or a pharmacologically acceptable salt thereof; wherein R is

(a) 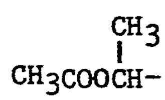

(b) 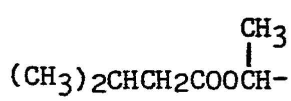

(c) 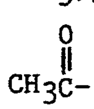 and

and $R_4$ is the same as in claim 1.

3. A compound of Claim 1 having the formula Ie

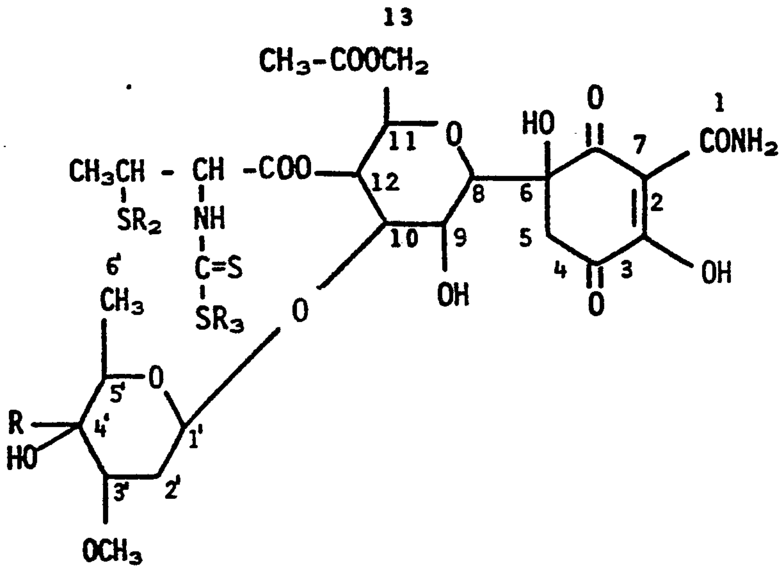

Ie

or a pharmaceutically acceptable salt thereof wherein

$R_2$ and $R_3$ are different and selected from the same group as in Claim 1 and R and $R_4$ are the same as in Claim 1.

4. A compound according to Claim 1 having the formula

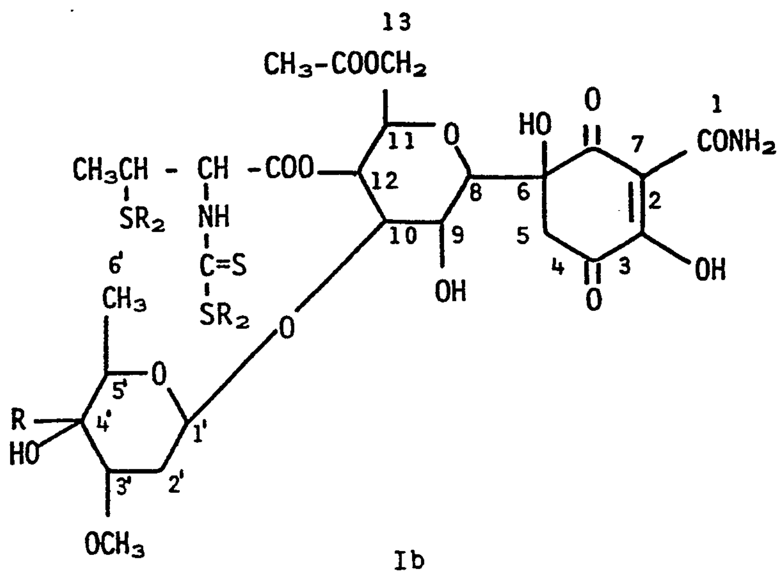

Ib

or a pharmacologically acceptable salt thereof wherein

R, $R_2$, and $R_3$, are the same as in Claim 1.

5. A process for preparing a compound of the formula Id:

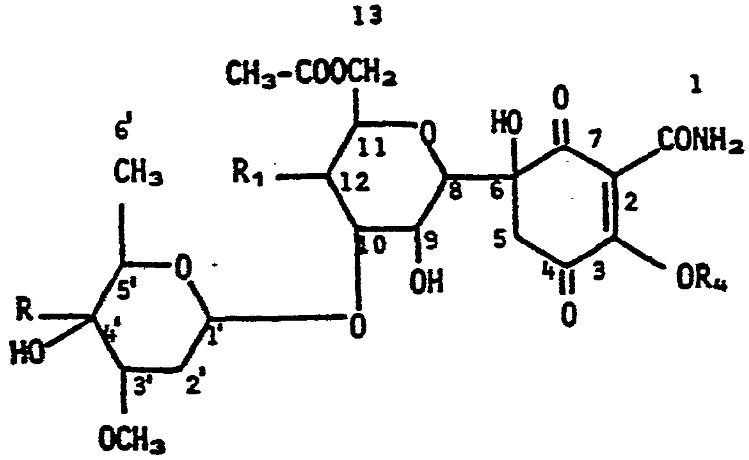

Id

wherein R is

(a) 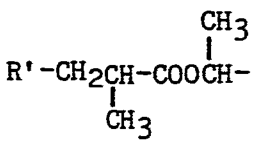

(b) 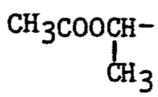

(c) 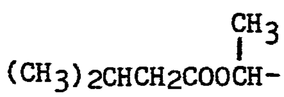

(d) 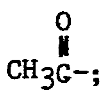; and

wherein R` is hydrogen or methyl;

wherein $R_1$ is

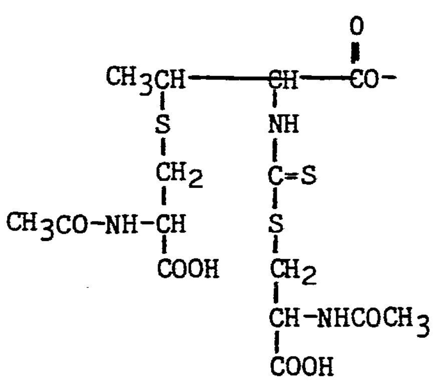

which comprises reacting a compound selected from the group consisting of Paulomycin A, B, $A_2$, D and E with N-acetyl-L-cysteine.

6. A process for preparing a compound of the formula Ib:

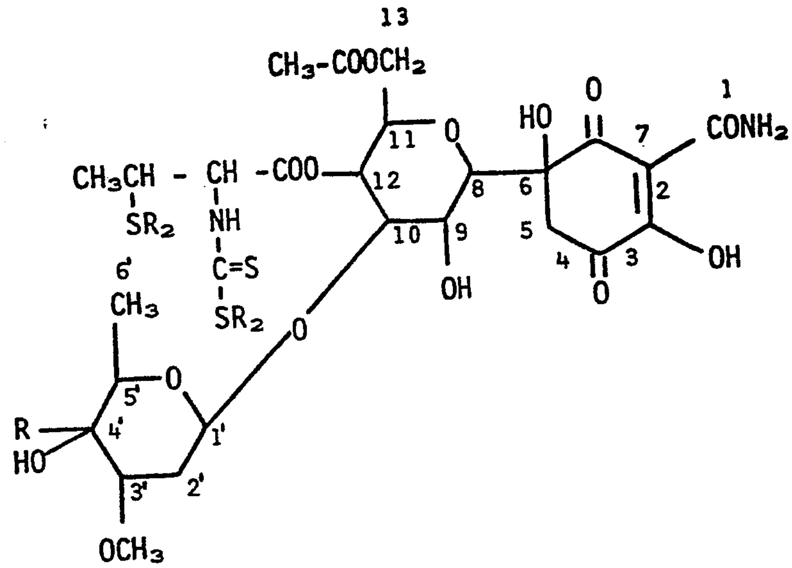

Ib

wherein R is

(a)
$CH_3$
$R'-CH_2CH-COOCH-$
$CH_2$

(b)
$CH_3$
$CH_3COOCH-$

(c)
$CH_3$
$(CH_3)_2CHCH_2COOCH-$

(d)
O
$CH_3C-$

wherein R' is hydrogen or methyl

wherein $R_2$ is

(a) $-CH_2COOH$,

(b) $CH_3CHCOOH$;

(c) COOH;
|
CH-
|
$CH_2$
|
COOH

(d) $-CH_2CHCOOH$;
|
$NH_2$

(e) $-CH_2CH_2CHCOOH$;
|
$NH_2$

(f) $H_2NCHCH_2CH_2CONHCHCONHCH_2COOH$;
| |
COOH $CH_2-$

(g)

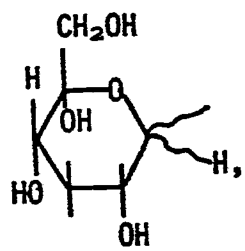

and, (h) $CH_2-$
|
CHOH
|
$CH_2OH$

which comprises reacting paulomycin A, $A_2$, B, D, or E with a compound selected from the following group: mercaptoacetic acid, 2-mercaptopropionic acid, thiomalic acid, cysteine, homocysteine, glutathione, thioglucose and thioglycerol.

7. A process for preparing a compound of the formula Ie:

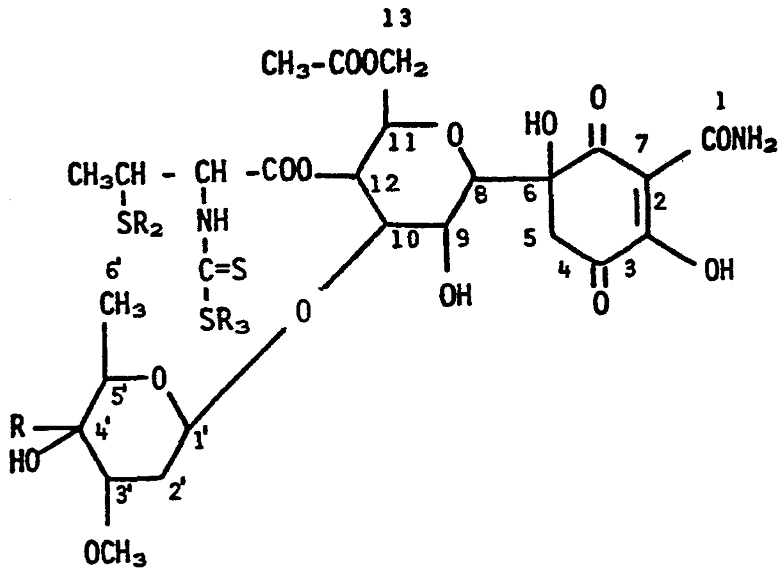

Ie

which comprises reacting a compound having the formula Ib

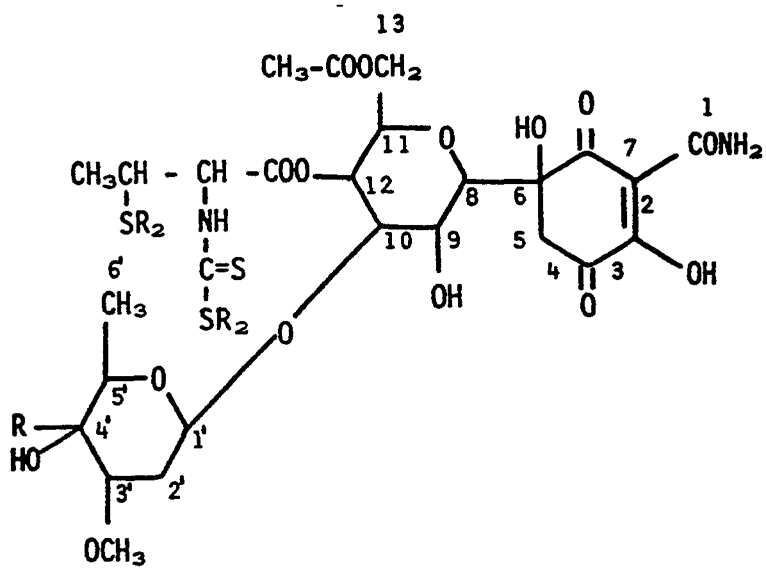

Ib

with a compound having the formula

$R_3SH$

wherein R is

(a) 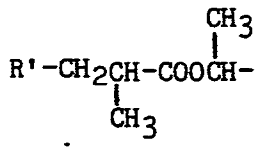

(b) 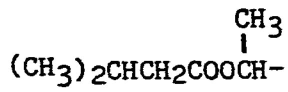

(c) 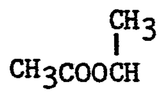

(d) 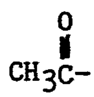

wherein R' is hydrogen or methyl;

$R_2$ and $R_3$ are different and are selected from the group consisting of

(a) $-CH_2COOH$,

(b) $CH_3CHCOOH$;

(c) 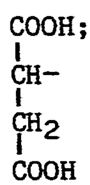

(d) 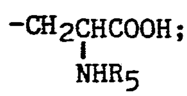

(e) 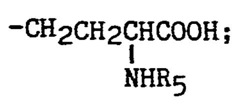

wherein $R_4$ is hydrogen or a pharmacologically acceptable salt and wherein $R_5$ is hydrogen or acetyl.

8. A process for preparing a compound of the formula Ic:

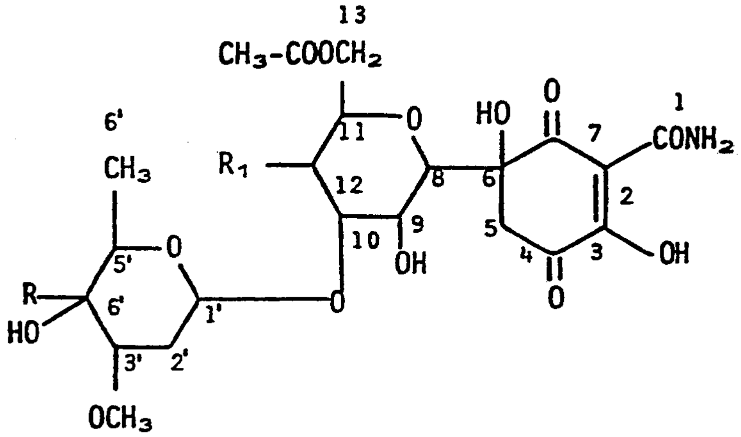

Ic

wherein R is

(a)
```
          CH3
          |
R'-CH2CH-COOCH-
      |
      CH3
```

(b)
```
     CH3
     |
CH3COOCH-
```

(c)
```
                CH3
                |
(CH3)2CHCH2COOCH-
```

(d)
```
    O
    ||
CH3C-
```

wherein $R_1$ is
```
  CH3CH-CHCOO-
HOCH2[CHOH]nCH2S   NHC=S
                   SCH2[CHOH]nCH2OH; and
```
wherein n is 3 or 4;

which comprises reacting paulomycin A, $A_2$, B, D or E with a 1-deoxy-1-thiopentitol or a 1-deoxy-1-thio-hexitol.

9. A process for preparing a pharmacologically acceptable salt of a compound of the formula I:

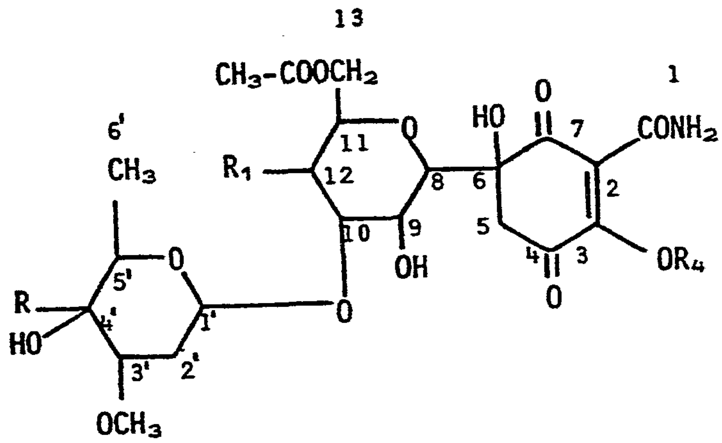

I

wherein R is

(a) 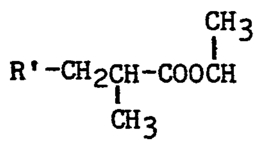

(b) 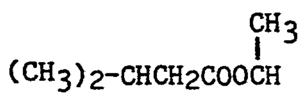

(c) 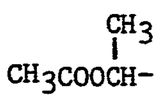

(d) 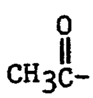

wherein R' is hydrogen or methyl;

wherein $R_1$ is

(a) 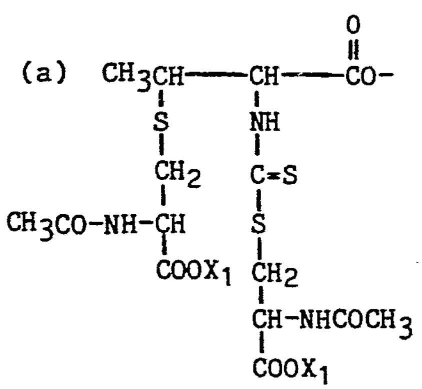

(b) 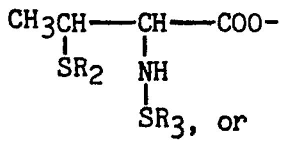, or

(c) 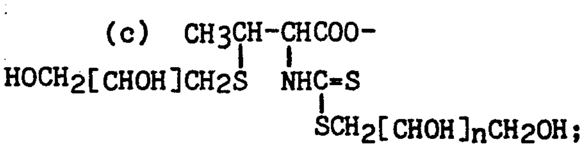

wherein $X_1$ is

(a) hydrogen,

(b) C1 to $C_{12}$ alkyl, branched or straight chain, or

(c) a pharmacologically acceptable cation;

wherein $R_2$ and $R_3$ are the same or different and are selected from the group

(a) $-CH_2COOH$,

(b) $CH_3CHCOOH$,

(c) 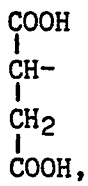

(d) 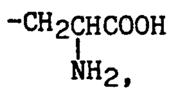

(e) 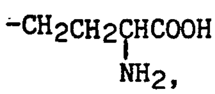

(f) 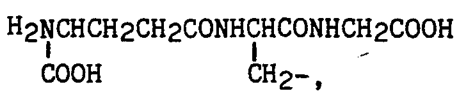

(g) 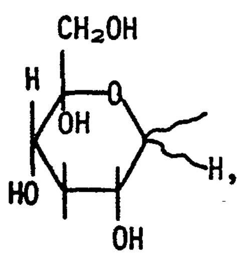

(h) $CH_2-$
|
CHOH
|
$CH_2OH$; and

(i) $CH_2-$
|
$[CHOH]_n$
|
$CH_2OH$

wherein n is 3 or 4;

(j) $-CH_2CH_2OH-$

with the proviso that one of $X_1$ or $R_4$ must be hydrogen; which comprises: reacting a compound of the formula I with an organic or inorganic base.

10. A Tribasic salt compound of claim 1 having the formula

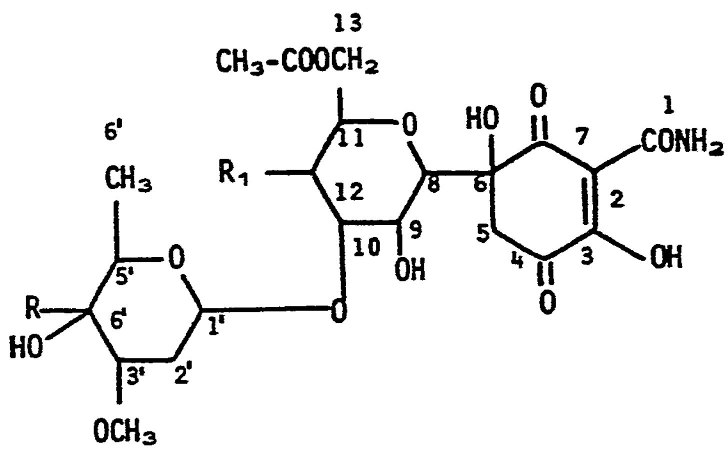

wherein R is

(a)
$$R'-CH_2CH(CH_3)-COOCH(CH_3)-$$

(b)
$$CH_3COOCH(CH_3)-$$

(c)
$$(CH_3)_2CHCH_2COOCH(CH_3)-$$

(d) 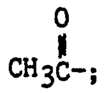; and

wherein R` is hydrogen or methyl;

wherein $R_1$ is

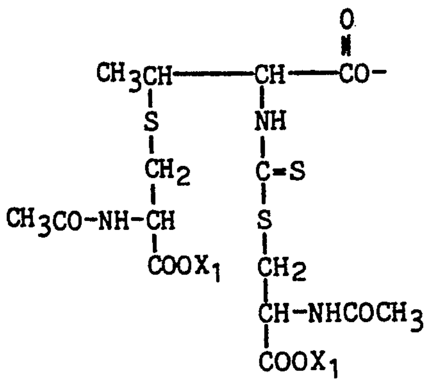

wherein X1 and $R_4$ are the same and are pharmacologically acceptable cations.